(19) 

**Europäisches Patentamt**
**European Patent Office**
**Office européen des brevets**

(11) **EP 2 822 450 B1**

(12) **FASCICULE DE BREVET EUROPEEN**

(45) Date de publication et mention de la délivrance du brevet:
**03.05.2023  Bulletin 2023/18**

(21) Numéro de dépôt: **13719889.1**

(22) Date de dépôt: **08.03.2013**

(51) Classification Internationale des Brevets (IPC):
*A61B 3/11* (2006.01)    *A61B 3/113* (2006.01)
*A61B 3/14* (2006.01)    *A61B 5/11* (2006.01)
*A61B 5/107* (2006.01)    *G02C 13/00* (2006.01)

(52) Classification Coopérative des Brevets (CPC):
**A61B 3/113; A61B 3/14; A61B 5/1121;**
**A61B 5/1128; G02C 13/003;** A61B 3/11;
A61B 5/1071; A61B 5/1072; A61B 5/1075;
A61B 5/1079

(86) Numéro de dépôt international:
**PCT/FR2013/000062**

(87) Numéro de publication internationale:
**WO 2014/128361 (28.08.2014 Gazette 2014/35)**

(54) **PROCEDE D'ESTIMATION D'UNE DISTANCE SEPARANT UNE PAIRE DE LUNETTES ET UN OIL DU PORTEUR DE LA PAIRE DE LUNETTES**

VERFAHREN ZUR SCHÄTZUNG DES ABSTANDES ZWISCHEN EINER BRILLE UND DEN AUGEN DES TRÄGERS DER BRILLE

METHOD FOR ESTIMATING A DISTANCE SEPARATING A PAIR OF GLASSES AND AN EYE OF THE WEARER OF THE PAIR OF GLASSES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **08.03.2012  FR 1200704**

(43) Date de publication de la demande:
**14.01.2015  Bulletin 2015/03**

(73) Titulaire: **Essilor International**
**94220 Charenton-le-Pont (FR)**

(72) Inventeurs:
 • HADDADI, Ahmed
  94220 Charenton-le-Pont (FR)
 • DELZERS, Jean
  94220 Charenton-le-Pont (FR)

(74) Mandataire: **Jacobacci Coralis Harle**
**32, rue de l'Arcade**
**75008 Paris (FR)**

(56) Documents cités:
**WO-A1-2008/132356    DE-A1-102009 004 383**

**Description**

DOMAINE TECHNIQUE AUQUEL SE RAPPORTE L'INVENTION

La présente invention concerne de manière générale la prise de mesures sur un sujet.

Elle trouve une application particulière, mais non exclusive, à la prise de mesures sur un porteur de lunettes en vue de la conception optique personnalisée de lentilles ophtalmiques correctrices adaptées à ce porteur.

Elle concerne plus particulièrement un procédé d'estimation d'une distance oeil-lunettes, séparant un plan caractéristique d'une paire de lunettes et un point caractéristique d'une tête d'un porteur de ladite paire de lunettes, comportant des étapes :

a) d'acquisition par un capteur d'images d'au moins deux images distinctes d'au moins une partie de la tête du porteur, sur lesquelles la tête du porteur présente des positions angulaires distinctes autour d'un axe de pivotement sensiblement parallèle audit plan caractéristique et transversal à l'axe optique du capteur d'images,

b) de détermination, pour chaque image acquise, d'un angle d'inclinaison de la tête du porteur par rapport au capteur d'image, mesuré entre l'axe optique du capteur d'images et un plan attaché à la tête du porteur qui est sensiblement parallèle audit axe de pivotement et sensiblement orthogonal audit plan caractéristique, et

c à e) de traitement des images pour estimer la distance oeil-lunettes recherchée.

Elle concerne également un dispositif d'estimation de la distance oeil-lunettes, qui comprend :

- au moins une source de lumière adaptée à éclairer la tête du porteur de lunettes,
- un capteur d'images présentant un axe optique sensiblement dirigé vers la tête du porteur et adapté à acquérir deux images de la tête du porteur sur lesquelles apparaît la paire de lunettes éclairée par la source de lumière et sur lesquelles la tête du porteur présente deux inclinaisons distinctes autour de l'axe de pivotement, et
- une unité de calcul adaptée à traiter lesdites images.

ARRIERE-PLAN TECHNOLOGIQUE

Lors de la conception d'une lentille ophtalmique correctrice, on cherche à prendre en compte un nombre important de paramètres géométrico-morphologiques individuels, dits de conception optique personnalisée, attachés au porteur et à la monture de lunettes sélectionnée, afin d'usiner la lentille de telle sorte qu'elle s'adapte au mieux au porteur.

Pour déterminer ces paramètres géométrico-morphologiques, l'opticien place la monture de lunettes sélectionnée sur le nez du porteur et réalise différentes opérations de mesure sur le porteur ainsi équipé. Ainsi peut-il notamment déterminer les demi-écarts pupillaires du porteur, c'est-à-dire les distances entre le plan de symétrie de la monture de lunettes et chacune des pupilles du porteur.

Ces mesures sont toutefois faussées lorsque le visage du porteur ne fait pas exactement face au capteur d'images, et qu'il présente ainsi un angle de lacet non nul par rapport au capteur d'images (l'angle de lacet correspondant à l'angle formé entre le plan sagittal de la tête du porteur est l'axe optique du capteur d'image).

On comprend en effet que lorsque le porteur a la tête tournée vers la droite, l'écart mesurable sur l'image acquise entre l'œil gauche et le centre de la monture sera plus grand que le demi-écart pupillaire gauche réel du porteur, tandis que l'écart mesurable entre l'œil droit et le centre de la monture sera inférieur au demi-écart pupillaire droit réel du porteur.

On constate plus précisément que lorsque l'angle de lacet est non nul, les mesures des demi-écarts pupillaires sont faussées d'environ 0,5 millimètre par degré d'angle de lacet.

Il convient alors de mesurer ces demi-écarts pupillaires non pas dans le plan de l'image acquise, mais dans le plan moyen de la monture de lunettes (qui est sensiblement orthogonal aux branches de la monture lorsqu'elles sont déployées).

Une solution consiste alors à déterminer les positions dans l'espace des centres de rotation des deux yeux du porteur pour en déduire les demi-écarts pupillaires. Autrement formulé, connaissant les positions des pupilles du porteur sur l'image acquise, il suffit de déterminer les distances séparant le plan moyen de la monture des centres de rotation des yeux du porteur pour en déduire les demi-écarts pupillaires du porteur.

Une méthode de détermination des distances « plan moyen - centres de rotation des yeux » est exposée dans le document WO 2008/132356. Elle consiste à équiper la monture de lunettes du porteur d'un système de repérage pour faciliter la localisation de la monture de lunettes sur l'image acquise, à demander au porteur de lunettes de tourner la tête en maintenant le regard sur un point fixe, à prendre deux clichés de la tête du porteur à deux instants différents, et à en déduire géométriquement les distances recherchées, compte tenu de la position de la monture de lunettes.

Cette méthode présente toutefois divers inconvénients.

Il est ainsi nécessaire de fixer avec soin le système de repérage sur la monture de lunettes, quelle que soit la forme de cette monture, ce qui peut s'avérer fastidieux et qui nécessite d'être mis en oeuvre par un opticien formé.

La tenue du système de repérage sur la monture peut en outre se révéler aléatoire, selon la forme de la monture de lunettes, et alors fausser les mesures.

On observe par ailleurs que la localisation du système de repérage est difficile à mettre en oeuvre automatiquement et présente un taux d'échec significatif, si bien que l'opticien est souvent forcé de localiser manuellement ce système de repérage sur l'image acquise.

Enfin, le système de repérage est peu esthétique, ce qui n'est pas flatteur pour le porteur de lunettes qui se voit dans un miroir et sur l'image acquise.

OBJET DE L'INVENTION

Afin de remédier aux inconvénients précités de l'état de la technique, la présente invention propose un nouveau procédé statistique d'estimation d'une distance séparant un point donné de la tête du porteur et un plan donné de la monture de lunettes.

Plus particulièrement, on propose selon l'invention un procédé d'estimation selon la Revendication 1, tel que défini dans l'introduction, dans lequel il est prévu entre autres des étapes

c) d'acquisition d'au moins deux valeurs simulées de la distance oeil-lunettes recherchée,

d) de calcul, pour chaque image acquise et avec chaque valeur simulée acquise, d'une distance morphologique entre le point caractéristique de la tête du porteur et le plan attaché à la tête du porteur, compte tenu dudit angle d'inclinaison, et

e) de sélection, parmi les valeurs simulées de la distance oeil-lunettes recherchée, de la valeur la plus proche de la distance oeil-lunettes du porteur, en fonction des distances tête-plan calculées à l'étape d).

Ainsi, grâce à l'invention, on utilise une méthode statistique dans laquelle on fait varier, sur chaque image, une valeur simulée pour obtenir une distance morphologique correspondante, et on sélectionne en fonction de l'ensemble de ces distances morphologiques la valeur simulée le plus proche de la valeur réelle.

Comme cela sera décrit en détail infra, l'étape d) sera basée sur un calcul trigonométrique itératif.

Ce calcul pourra bien entendu être réalisé autrement. Il pourrait par exemple être réalisé au moyen d'un calcul trigonométrique simple, en prenant en compte un nombre important de variables (par exemple la largeur et la hauteur préalablement mesurées de chaque cercle de la monture de lunettes).

Comme cela sera décrit en détail infra, l'étape e) sera préférentiellement opérée en sélectionnant la valeur simulée pour laquelle l'écart-type entre les distances tête-plan calculées à l'étape b) est le plus faible.

Ce calcul pourra bien entendu être réalisé autrement. Il pourrait par exemple être réalisé au moyen d'une fonction mathématique non pas d'écart-type mais plutôt de variance.

Ici, on définit l'expression "point caractéristique de l'œil" comme tout point repérable ou distinctif de l'œil.

Il pourra s'agir du centre de rotation de l'oeil. Il pourrait bien entendu s'agir en variante de la macula ou du centre de la pupille de l'oeil.

La "distance morphologique" mentionnée ci-dessus se définit alors comme la distance entre ce point caractéristique de l'œil du porteur et le plan attaché à la tête du porteur (par exemple le plan sagittal, ou le plan de Francfort).

La "position angulaire" de la tête du porteur se définit comme la position angulaire de la tête du porteur par rapport au capteur d'images, autour d'un axe de pivotement transversal à l'axe optique du capteur d'images. Elle correspond alors à l'angle formé entre l'axe optique du capteur d'images et le plan attaché à la tête du porteur (par exemple le plan sagittal, ou le plan de Francfort).

Le « plan caractéristique de la paire de lunettes » se définit comme un plan déterminable de la paire de lunettes. Il pourra par exemple s'agir du plan qui passe par le sommet du pontet de la monture et qui est sensiblement orthogonal aux branches déployées de la monture. Il pourrait en variante s'agir du plan qui passe par les sommets des faces avant (ou arrière) des lentilles et qui est sensiblement orthogonal aux branches déployées de la monture.

La "distance oeil-lunette" se définit quant à elle comme la distance entre le plan caractéristique de la paire de lunettes et le point caractéristique de l'œil du porteur.

D'autres caractéristiques avantageuses et non limitatives du procédé conforme à l'invention sont les suivantes :

- à l'étape a), ledit axe de pivotement est confondu avec l'axe longitudinal de la tête du porteur, et à l'étape b), le plan attaché à la tête du porteur est parallèle au plan sagittal de la tête du porteur et ledit angle d'inclinaison est un angle de lacet ;
- le point caractéristique de la tête du porteur est le centre de rotation de l'un des deux yeux du porteur, le plan attaché à la tête du porteur est confondu avec le plan sagittal de la tête du porteur, et chaque distance morphologique calculée à l'étape d) est un demi-écart pupillaire supposé du porteur calculé en fonction de la position sur l'image acquise de la position d'un reflet cornéen réfléchi par la cornée dudit oeil du porteur ;

- la position du plan attaché à la tête du porteur est obtenue en repérant la position de la paire de lunettes sur chacune des images acquises ;
- à l'étape e), on détermine, pour chaque valeur simulée, un taux de dispersion des distances morphologiques associées à cette valeur simulée, et la valeur simulée sélectionnée est celle pour laquelle les distances morphologiques sont les moins dispersées ;
- le taux de dispersion des distances morphologiques est un écart-type ;
- il est prévu une étape supplémentaire d'estimation du demi-écart pupillaire du porteur, au cours de laquelle est calculée la moyenne des distances morphologiques associées à la valeur simulée sélectionnée ;
- le dispositif comportant une source de lumière, à l'étape b), ledit angle d'inclinaison est déterminé en fonction de la position, sur chaque image, des reflets générés par la source de lumière et réfléchis par les deux lentilles de la paire de lunettes ;
- ladite source de lumière est infrarouge et ledit capteur d'images est adapté à acquérir des images infrarouges ;
- le plan caractéristique de la paire de lunettes est parallèle au plan moyen des contours des lentilles de la paire de lunettes ;
- ledit angle d'inclinaison est obtenu en installant sur la paire de lunettes un élément de repérage ayant une géométrie connue, et en déterminant la position et la forme dudit élément de repérage sur chaque image acquise ;
- on acquiert une dimension caractéristique de ladite paire de lunettes et ladite distance oeil-lunettes du porteur est mise à l'échelle en fonction de ladite dimension caractéristique acquise.

L'invention concerne également un dispositif d'estimation d'une distance oeil-lunettes, entre un plan caractéristique d'une paire de lunettes et un point caractéristique d'une tête d'un porteur de ladite paire de lunettes, défini dans la Revendication 11 et qui comprend entre autres

- au moins une source de lumière adaptée à éclairer la tête du porteur,
- un capteur d'images présentant un axe optique sensiblement dirigé vers la tête du porteur et adapté à acquérir au moins deux images de la tête du porteur sur lesquelles apparaît la paire de lunettes éclairée par la source de lumière et sur lesquelles la tête du porteur présente des inclinaisons distinctes autour d'un axe de pivotement sensiblement parallèle audit plan caractéristique et transversal à l'axe optique du capteur d'images, et
- une unité de calcul adaptée à traiter lesdites images afin de :

  - déterminer, pour chaque image acquise, un angle d'inclinaison de la tête du porteur par rapport au capteur d'image, mesuré entre l'axe optique du capteur d'images et un plan attaché à la tête du porteur qui est sensiblement parallèle audit axe de pivotement et sensiblement orthogonal audit plan caractéristique,
  - acquérir au moins deux valeurs simulées de la distance oeil-lunettes recherchée,
  - calculer, pour chaque image acquise et avec chaque valeur simulée acquise, une distance morphologique entre le point caractéristique de la tête du porteur et le plan attaché à la tête du porteur, compte tenu dudit angle d'inclinaison, et
  - sélectionner, parmi les valeurs simulées de la distance oeil-lunettes recherchée, la valeur la plus proche de la distance oeil-lunettes du porteur, en fonction des distances tête-plan calculées à l'étape d).

DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.
Sur les dessins annexés :

- la figure 1 est une vue schématique en perspective de la tête d'un porteur de lunettes ;
- la figure 2 est une vue schématique de dessus de la tête du porteur de lunettes de la figure 1 ;
- la figure 3 est un schéma sur lequel apparaissent les yeux du porteur de lunettes de la figure 1 et un capteur d'images ;
- la figure 4 est une vue schématique en perspective d'un dispositif adapté à mettre en oeuvre le procédé selon l'invention ;
- les figures 5 à 7 sont des représentations de trois images acquises par le capteur d'images de la figure 3 ; et
- la figure 8 est une vue schématique de dessus de la tête du porteur de lunettes de la figure 1.

Dans la description qui va suivre, certaines références auront un numéro suivi de la lettre G ou D. Ces références désigneront alors respectivement des éléments situés à gauche ou à droite par rapport au porteur de lunettes. Les yeux gauche et droit du porteur seront ainsi respectivement référencés 20G et 20D.

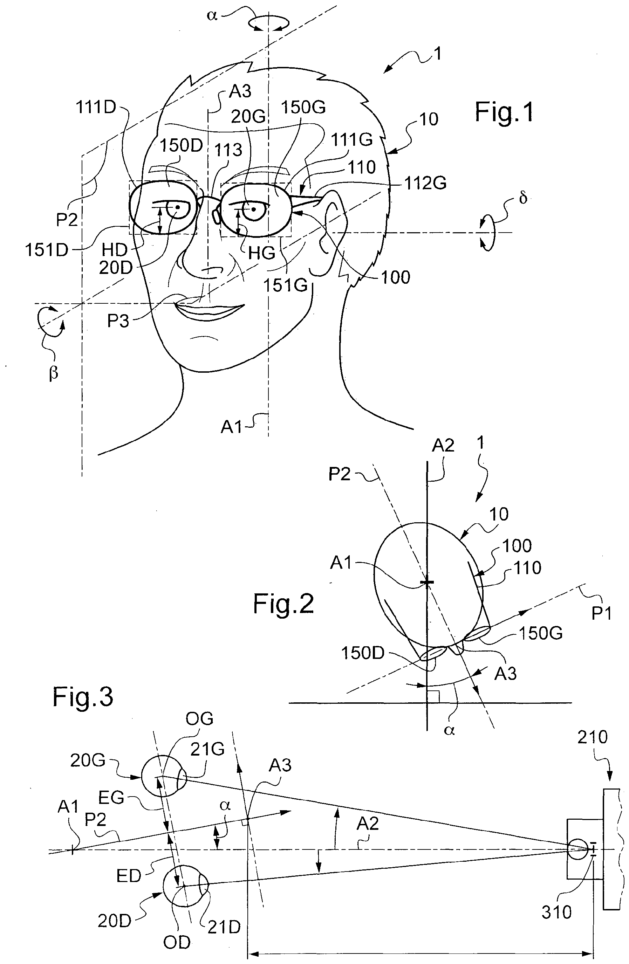

Sur la figure 1, on a représenté la tête 10 d'un individu 1 portant une paire de lunettes 100. Cet individu sera désigné dans la suite de cet exposé comme le « porteur ».

Dans cet exposé, on considérera également un autre individu, qui sera désigné comme le « vendeur », et qui aidera le porteur 10 à choisir une paire de lunettes 100 et à mesurer les données nécessaires à la fabrication des lentilles ophtalmiques (non représentées) en vue de leur commande et de leur montage dans la monture de lunettes 100 sélectionnée par le porteur.

Grâce à l'automatisation des mesures, ce vendeur ne sera pas nécessairement un opticien.

Préalablement aux mesures, le porteur choisira une paire de lunettes 100 parmi les paires de lunettes mises à disposition par le vendeur.

Lors des mesures, le porteur portera cette paire de lunettes 100.

Il sera positionné dans une configuration assise ou debout, avec sa tête 10 sensiblement droite et dirigée vers un capteur d'images 210 (voir figure 3).

Le vendeur demandera par ailleurs au porteur 1 de regarder une cible 310 située à proximité du capteur d'images 210 (voir figure 3). Les centres des deux pupilles 21G, 21D et les centres de rotation OD, OG des deux yeux 20G, 20D du porteur 1 seront donc respectivement alignés avec la cible 310.

Sur la figure 1, on observe que la paire de lunettes 100 choisie par le porteur 1 est du type cerclée (en variante, elle pourrait bien entendu être d'une autre sorte, telle que semi-cerclée ou percée).

Cette paire de lunettes 100 comporte une monture de lunettes 110, avec deux cercles 111G, 111D (ou entourages) dans lesquels sont emboîtées deux lentilles de présentation 150G, 150D (destinées à être remplacées par les lentilles ophtalmiques adaptées à l'acuité visuelle du porteur).

Les deux cercles 111G, 111D sont reliés l'un à l'autre par un pont ou pontet 113 équipé de deux plaquettes nasales reposant sur le nez du porteur. Ils sont également chacun équipés d'une branche 112G, 112D reposant sur les oreilles correspondantes du porteur 1. Ces branches 112G, 112D s'étendent chacune de manière rectiligne sur leur majeure partie, selon un axe longitudinal, et sont recourbées à leurs extrémités.

Chacun des deux cercles 111G, 111D de la monture de lunettes 110 présente, en creux dans son flanc intérieur, une rainure communément appelée drageoir dans laquelle est emboîté un biseau qui s'étend en saillie de la tranche de la lentille de présentation 150G, 150D correspondante.

Comme le montre la figure 2, on définit ici par rapport à la monture de lunettes 110 un plan moyen P1 qui passe au plus près de l'ensemble des points de l'arête de fond des drageoirs des deux cercles 111G, 111D.

Ce plan moyen P1 est incliné par rapport au plan passant par les axes longitudinaux des branches 112G, 112D d'un angle appelé « angle pantoscopique ». L'angle pantoscopique moyen d'une monture de lunettes est de l'ordre de 10 degrés.

Comme le montre la figure 1, sur chaque image du porteur 1 acquise par le capteur d'images 210, on peut repérer les cercles 111G, 111D de la monture de lunettes 110 au moyen de deux cadres appelés « cadres boxing » 151G, 151D.

Ces cadres boxing 151G, 151D sont définis comme les rectangles circonscrits aux cercles 111G, 111D de la monture de lunettes 110 (ou aux lentilles de présentation 150G, 150D), dont deux des côtés sont verticaux et dont les deux autres sont horizontaux.

On définit alors sur chaque image un axe remarquable A3 de la monture de lunettes 100, comme étant l'axe qui est parallèle aux côtés verticaux des cadres boxing 151G, 151D, et qui est situé à égale distance de ces derniers.

Comme le montre la figure 1, on définit par rapport au porteur 1 en position de mesure un plan de Francfort P3 comme étant le plan passant par les points orbitaires inférieurs et le porion du porteur (le porion étant le point du crâne le plus élevé du conduit auditif, qui correspond au tragion de l'oreille). En position de mesure, ce plan de Francfort P3 sera donc sensiblement horizontal.

On définit également un plan sagittal P2 comme étant le plan orthogonal au plan de Francfort P3 et à l'axe passant par les centres de rotation OG, OD des deux yeux 20G, 20D du porteur 1, passant par l'arête du nez du porteur. Lors des mesures, ce plan sagittal P2 sera donc sensiblement vertical. Sa position sera déduite non pas de la position du nez du porteur 1, mais plutôt en fonction de la position de la paire de lunettes 100 portée par le porteur 1.

On définit par rapport à la tête 10 du porteur 1 un axe longitudinal A1 comme étant orthogonal au plan de Francfort P3, compris dans le plan sagittal P2, et correspondant à l'axe de rotation de la tête 10 du porteur 1 lorsque ce dernier fait un mouvement de tête de droite à gauche (c'est-à-dire lorsque le porteur fait Non de la tête). En position de mesure, cet axe longitudinal A1 sera sensiblement vertical.

On définit également un axe frontal A4 comme étant l'axe d'intersection entre le plan de Francfort P3 et le plan sagittal P2.

On définit aussi un axe transversal A5 comme étant l'axe perpendiculaire aux axes longitudinal A1 et frontal A4.

En position de mesure, ces deux axes frontal et transversal A4, A5 seront sensiblement horizontaux.

Comme le montre la figure 3, les demi-écarts pupillaires EG, ED du porteur 1 sont définis comme les distances séparant le plan sagittal P2 des centres de rotation OG, OD des deux yeux 20G, 20D du porteur 1.

Comme le montre la figure 1, les hauteurs pupillaires HG, HD du porteur sont quant à elles définies comme les distances séparant, sur chaque image acquise, les centres de rotation OG, OD des yeux 20G, 20D du porteur 1 (qui sont en pratique confondus sur l'image avec les centres des pupilles 21G, 21D du porteur 1) et le bord inférieur du cadre boxing 151G, 151D correspondant.

Tel que représenté sur la figure 3, on définit par ailleurs par rapport au capteur d'images 210 un plan horizontal et un plan vertical, dont l'intersection est confondue avec l'axe optique A2 du capteur d'images 210.

Idéalement, on cherche alors à placer la tête 10 du porteur 1 de manière que son plan de Francfort P3 se confonde avec le plan horizontal du capteur d'images 210 et que son plan sagittal P2 se confonde avec le plan vertical du capteur d'images 210.

En pratique, on constate généralement un léger décalage entre ces plans, susceptible de fausser les mesures.

Ce décalage peut être mesuré au moyen de trois angles de roulis $\beta$, de tangage $\delta$ et de lacet $\alpha$, qui correspondent aux trois mobilités de pivotement de la tête 10 du porteur 1.

L'angle de lacet $\alpha$ sera ainsi défini comme l'angle de pivotement de la tête 10 du porteur 1 autour de l'axe longitudinal A1, entre la position idéale de la tête 10 du porteur (face à l'axe optique A2) et la position réelle de la tête 10 du porteur. Cet angle de lacet $\alpha$ sera mesuré dans le plan horizontal du capteur d'images 210.

L'angle de roulis $\beta$ sera défini comme l'angle de pivotement de la tête 10 du porteur 1 autour de l'axe frontal A4, entre la position idéale de la tête 10 du porteur et la position réelle de la tête 10 du porteur. Cet angle de roulis $\beta$ sera facilement mesurable sur chaque image acquise, en fonction de l'inclinaison de l'axe remarquable A3.

L'angle de tangage $\delta$ sera défini comme l'angle de pivotement de la tête 10 du porteur 1 autour de l'axe transversal A5, entre la position idéale de la tête 10 du porteur (face à l'axe optique A2) et la position réelle de la tête 10 du porteur. Cet angle de tangage $\delta$ sera mesuré dans le plan vertical du capteur d'images 210.

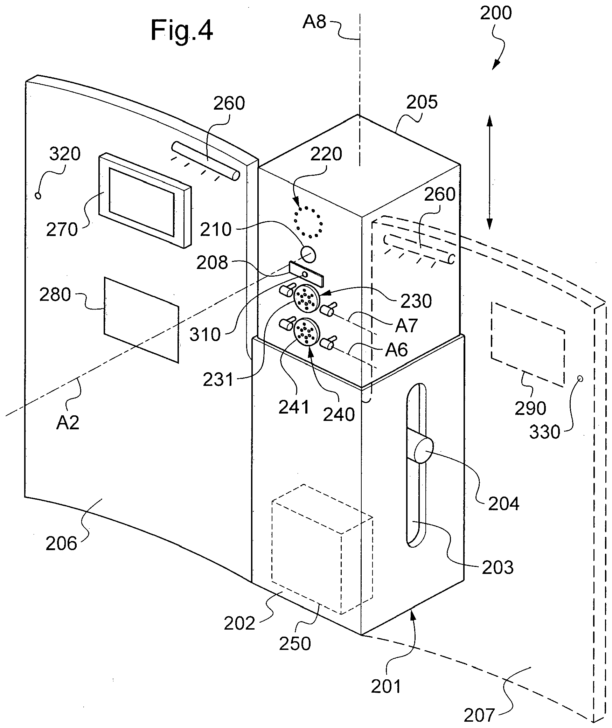

Sur la figure 4, on a représenté le dispositif 200 qui permet de mettre en oeuvre le procédé selon l'invention et qui, plus généralement, permet de mesurer et d'acquérir les données nécessaires à la commande des lentilles ophtalmiques à monter dans la monture de lunettes 110 sélectionnée par le porteur 1, en lieu et place des lentilles de présentation 150G, 150D.

Ce dispositif se présente sous la forme d'un kiosque à lunettes 200, faisant simultanément office de présentoir de montures de lunettes et de centre de mesure.

Il comporte à cet effet au moins un châssis 201, une source de lumière 220, 230, 240, un capteur d'images 210 et une unité de calcul 250.

De manière préférentielle, la source de lumière 220, 230, 240 émet dans l'infrarouge et le capteur d'images 210 capte des images infrarouges.

Le domaine de l'infrarouge qui sera ici utilisé est le proche infrarouge, dont les longueurs d'onde sont comprises entre 780 et 1400 nm.

L'utilisation d'une lumière infrarouge présente en effet plusieurs avantages. Elle permet notamment de s'affranchir des reflets parasites, provenant de la lumière extérieure ou de la lumière intérieure qui se réfléchit sur les lentilles de présentation 150G, 150D de la paire de lunettes 100. Elle permet aussi d'éviter au porteur 1 d'être ébloui lors des mesures. Elle permet enfin de voir les yeux du porteur 1 sur les images acquises par le capteur d'images 210, même lorsque les lentilles de présentation 150D, 150G sont teintées.

Le châssis pourrait être réalisé d'une seule pièce, de telle manière que le capteur d'images et les sources de lumière soient immobiles par rapport au sol sur lequel le kiosque à lunettes repose. Dans cette éventualité, il serait nécessaire de prévoir un tabouret réglable en hauteur, de manière à pouvoir placer le porteur de telle sorte que sa tête entre dans le champ du capteur d'images.

Au contraire, dans le mode de réalisation du kiosque 200 représenté sur la figure 4, le châssis 201 comprend un pied 202 qui est posé sur le sol, et une coulisse 205 qui est montée mobile en translation sur le pied 202 suivant un axe vertical A8 et qui supporte les sources de lumière 220, 230, 240 et le capteur d'images 210.

La position du capteur d'images 210 est ainsi réglable en hauteur en fonction de la taille du porteur 1.

Le pied 202 présente plus précisément une forme de parallélépipède creux, allongé suivant l'axe vertical A8 et de section horizontale carrée.

Ce pied 202 présente une extrémité inférieure fermée par une paroi posée au sol, et une extrémité supérieure ouverte par laquelle s'engage la coulisse 205.

Cette coulisse 205 présente à cet effet une forme parallélépipédique creuse, avec une section horizontale de dimensions extérieures égales aux dimensions intérieures du pied 202, ce qui lui permet de coulisser dans le pied 202.

Cette coulisse 205 présente une extrémité inférieure ouverte vers l'intérieur du pied 202, et une extrémité supérieure fermée par une paroi plane.

La coulisse 205 présente, sur deux côtés opposés, deux ergots 204 en saillie engagés dans deux trous oblongs verticaux 203 pratiqués dans deux côtés opposés du pied 202, pour permettre le guidage de la coulisse 205 en translation suivant l'axe vertical A8, entre deux positions de butée haute et basse.

Il est par ailleurs prévu des moyens d'actionnement motorisés (non représentés) permettant de monter ou descendre la coulisse 205 dans le pied 202, à la hauteur souhaitée.

Il est également prévu des moyens de détermination (non représentés) de la hauteur de la coulisse 205 dans le pied 202 (par exemple une roue codeuse associée à une crémaillère).

Le châssis 200 présente par ailleurs deux ailes latérales 206, 207 qui bordent sa face avant tournée vers le porteur 1.

Ces deux ailes latérales 206, 207 sont formées par des parois verticales légèrement incurvées vers l'avant et sont montées sur charnière sur les deux côtés opposés du pied 202 où sont pratiqués les trous oblongs verticaux 203.

Les faces avant de ces ailes 206, 207 sont par ailleurs équipées de crochets (non représentés) sur lesquels reposent les montures de lunettes parmi lesquelles le porteur fait son choix.

Sur la figure 4, on observe que la coulisse 205 porte au moins deux (ici trois) sources de lumière 220, 230, 240 infrarouges adaptées à éclairer la tête 10 du porteur 1 en position de mesure.

Ces sources de lumière 220, 230, 240 sont préférentiellement réparties de part et d'autre du capteur d'image, dans le plan vertical du capteur d'images 210.

Ces trois sources de lumière 220, 230, 240 sont ici montées fixes en translation sur la coulisse 205 et sont formées d'une pluralité de diodes électroluminescentes (LED).

L'une des sources de lumière, appelée source principale 220, est située à faible distance du capteur d'images 210 (c'est-à-dire ici à moins de 10 centimètres du capteur d'images). Elle est composée d'une pluralité de LED réparties sur un cercle. Ces LED sont directement fixées à la coulisse 205. Telle que représentée sur la figure 4, cette source principale 220 est située au-dessus du capteur d'images 210.

Les deux autres sources de lumière, appelées sources secondaires 230, 240, sont situées l'une au-dessus de l'autre, en dessous du capteur d'images 210. Elles sont chacune composées d'un nombre de LED égal à celui de la source principale 220, réparties sur deux cercles concentriques. Elles émettent une lumière d'intensité inférieure à celle de la lumière émise par la source principale 220. Les intensités lumineuses émises par les sources secondaires 230, 240 sont, au même titre que celle émise par la source principale 220, réglables.

Ces deux sources secondaires 230, 240 sont fixées sur des socles 231, 241 montés mobiles en rotation sur la coulisse 205 autour de deux axes de rotation A6, A7 horizontaux distincts. Grâce à cette mobilité, il est possible de diriger manuellement ces sources secondaires 230, 240 vers la tête 10 du porteur 1.

Les trois sources de lumière 220, 230, 240 sont plus précisément conçues pour former des reflets distincts sur les lentilles de présentation 150G, 150D de la paire de lunettes 100 portée par le porteur 1.

Ainsi, lorsque toutes les sources de lumière 220, 230, 240 se reflètent sur les deux lentilles de présentation 150G, 150D, le capteur d'images 210 peut observer six reflets-verres.

L'utilisation de trois sources de lumière 220, 230, 240 permet au capteur d'image 201 de voir au moins une partie de ces reflets, quel que soit l'angle de tangage $\delta$ de la tête du porteur 1. On comprend en effet que lorsque le porteur 1 incline la tête vers le bas, les reflets vus par le capteur d'images 210 remontent sur les lentilles de présentation 150G, 150D jusqu'à en sortir.

Les trois sources de lumière 220, 230, 240 forment par ailleurs un unique reflet sur chaque oeil du porteur 1, appelé reflet cornéen.

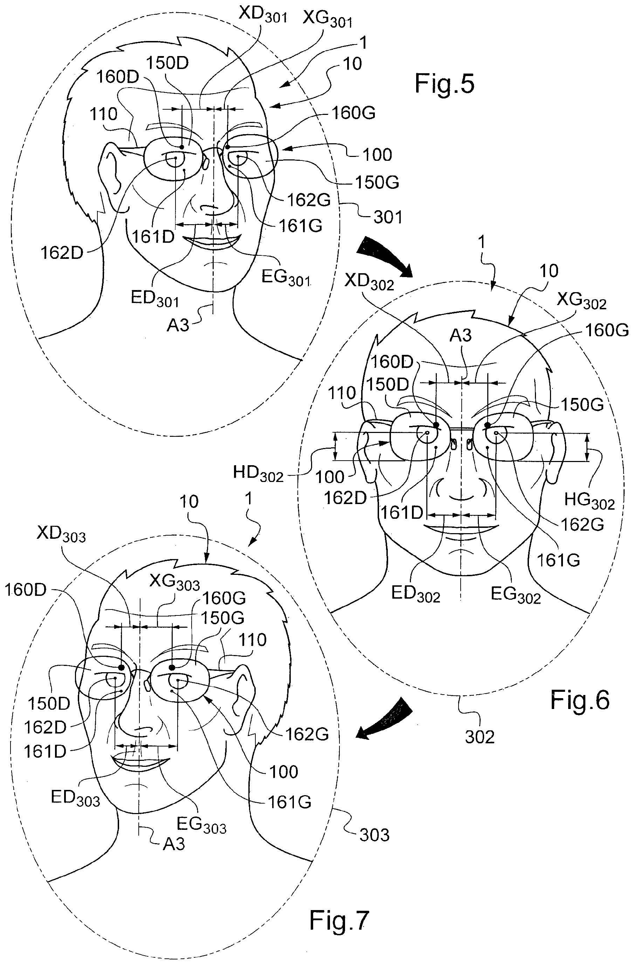

Sur la figure 6, on a représenté une situation dans laquelle seule deux des trois sources de lumière 220, 230, 240 se reflètent sur les deux lentilles de présentation 150G, 150D. On observe ainsi quatre reflets-verres 160G, 160D, 161G, 161D générés par la source principale 220 et l'une des sources secondaires 230 sur les deux lentilles de présentation 150G, 150D, et deux reflets cornéens 162G, 162D. La troisième source de lumière 240 est en revanche trop basse, eu égard à l'angle de tangage $\delta$ de la tête du porteur 1, pour générer des reflets sur les lentilles de présentation 150G, 150D.

Bien entendu, en variante, on pourrait prévoir que le kiosque ne comporte qu'une seule et unique source de lumière infrarouge.

Cette dernière pourrait être montée fixe à une position prédéterminée par rapport au capteur d'images. Il sera alors préférable d'utiliser une source de lumière de taille assez importante pour générer un reflet cornéen détectable. Cette source de lumière devra par ailleurs être placée de telle sorte que les deux reflets-verres qu'elle génère soient sensiblement situés à mi-hauteurs des lentilles de présentation 150G, 150D (c'est-à-dire sur l'axe passant par les centres des deux cadres boxing de la monture) lorsque la tête 10 du porteur 1 est idéalement placée (plan de Francfort P3 confondu avec le plan horizontal du capteur d'images 210 et plan sagittal P2 confondu avec le plan vertical du capteur d'images 210), compte tenu de l'angle pantoscopique moyen des montures.

Il conviendra alors de vérifier pendant les mesures que l'angle de tangage de la tête du porteur reste réduit, de sorte que le capteur d'image puisse voir les reflets de cette source de lumière sur les lentilles de présentation.

Selon une autre variante, on pourra prévoir que cette unique source de lumière soit montée mobile en translation verticale par rapport au capteur d'images, pour compenser l'angle de tangage de la tête du porteur.

Tel que le montre la figure 4, le kiosque 200 comporte ici un seul et unique capteur d'images 210, d'axe optique A2 horizontal.

Ce capteur d'image est ici formé par une caméra 210 adaptée à acquérir des images dans le proche infrarouge et dans le visible.

Cette caméra présente ici les références suivantes : Sony® FCB-EX490. Elle est équipée d'un filtre infrarouge basculant entre une position normale dans laquelle ce dernier filtre les infrarouges pour que la caméra 210 puisse acquérir une image dans le domaine du visible (appelée dans la suite « image-visible »), et une position escamotée dans laquelle il laisse les infrarouges passer jusqu'au capteur qui peut alors acquérir une image dans le domaine infrarouge (appelée dans la suite « image-infrarouge »).

Cette caméra est ainsi adaptée à acquérir des images-infrarouges du porteur sur lesquelles apparaissent nettement les reflets-verres et les reflets cornéens, ainsi que des images-visibles du porteur permettant à ce dernier de vérifier que la paire de lunettes 100 sélectionnée lui sied bien.

Bien entendu, en variante, il sera possible de prévoir non pas une seule caméra, mais deux caméras distinctes, l'une adaptée à acquérir des images-infrarouges et l'autre adaptée à acquérir des images-visibles.

Ici, la paroi avant de la coulisse 205 présente une fenêtre fermée par un miroir sans tain, à l'arrière duquel se trouve la caméra 210. La caméra 210 est ainsi invisible depuis l'extérieur du kiosque 200, mais elle demeure adaptée à acquérir des images des individus placés à l'avant du kiosque 200.

La caméra 210 est alors installée dans le kiosque 200 de telle manière que son objectif soit situé au contact ou à proximité de la face arrière de ce miroir sans tain.

L'objectif de la caméra 210 est par ailleurs entouré d'une paroi latérale opaque, permettant d'éviter que des reflets parasites n'apparaissent sur les images acquises.

En l'espèce, l'ensemble de la caméra est ici logé dans une boîte opaque qui est ouverte vers l'avant par une ouverture au travers de laquelle émerge l'objectif, cette boîte étant située au contact de la face arrière du miroir sans tain.

Le kiosque à lunettes 200 comporte par ailleurs au moins une cible 310 située sur le côté et à distance réduite de l'objectif de la caméra 210, de telle sorte qu'elle est visible par le porteur 1 en position de mesure.

Cette cible 310 comporte ici une fenêtre 208 pratiquée dans la paroi de la coulisse 205, entre l'objectif de la caméra 210 et les sources secondaires 230, 240, au travers de laquelle une LED est visible. En pratique, cette LED est ici située dans le pied 202 du châssis 200, et est réfléchie vers la fenêtre 208 au moyen d'un jeu de miroir.

Cette cible 310 permet d'attirer le regard du porteur 1 vers l'objectif de la caméra 210 lors des mesures.

Il est également prévu deux cibles supplémentaires 320, 330, situées sur les bords extérieurs de chacune des ailes 206, 207 du châssis 200, dans le plan horizontal de la caméra 210.

Comme cela sera bien exposé dans la suite de ce texte, ces cibles supplémentaires 320, 330 permettront d'attirer séquentiellement le regard du porteur 1 vers l'un des côtés du kiosque 200 puis l'autre, afin de déterminer si ce porteur 1 présente une propension à détourner le regard en bougeant plutôt la tête 10 ou les yeux 20G, 20D.

Il est par ailleurs prévu deux néons 260, respectivement positionnés sur les bords supérieurs des deux ailes 206, 207 du châssis 200, qui éclairent la tête 10 du porteur 1 de telle sorte que les images-visibles acquises par la caméra 210 soient bien exposées.

Il est aussi prévu dans l'une de ces deux ailes 206 des moyens d'affichage 270 et des moyens d'impression 280 des images-visibles acquises.

Les moyens d'affichage sont ici constitués par un écran tactile 270 fixé sur la face avant de l'aile 206, de manière à être visible par le porteur 1 en position de mesure.

Les moyens d'impression sont quant à eux conçus pour imprimer des fiches récapitulatives des données de commande des lentilles ophtalmiques, sur lesquelles apparaissent les photos des porteurs.

Il est par ailleurs prévu des moyens pour scanner les prescriptions du porteur 1.

Ici, les moyens d'impression et les moyens pour scanner sont confondus et sont constitués par une unique imprimante multifonction 280 couleur, située dans un logement prévu en creux dans la face avant de l'aile 206 du châssis 200.

Bien entendu, en variante, il serait également possible de prévoir deux appareils distincts, l'un pour imprimer les fiches récapitulatives et l'autre pour scanner les prescriptions.

Il est enfin prévu sur l'autre des deux ailes 207 un moyen d'acquisition d'au moins une dimension de la paire de lunettes 100. Les images acquises par la caméra 210 ne permettent en effet pas de déterminer les dimensions de la paire de lunettes 100 du porteur 1. Il est alors nécessaire de mettre à l'échelle les images acquises.

Les moyens d'acquisition 290 précités peuvent alors se présenter sous diverses formes.

De manière préférentielle, ils comportent un lecteur de code-barres 290 connecté à une base de données dont chaque enregistrement est associé à une référence de montures de lunettes et comprend un identifiant et des données relatives à cette référence de montures de lunettes.

En l'espèce, l'identifiant de chaque enregistrement est formé par le numéro du code-barres qui est assigné à la référence de montures de lunettes.

Les données mémorisées dans chaque enregistrement comportent quant à elles ici :

- la largeur totale des montures de lunettes de la référence considérée, mesurée entre les deux branches 112D, 112G,
- le rapport entre la hauteur et la largeur des cadres boxing 151G, 151D des cercles de ces montures de lunettes, et
- l'écart entre ces deux cadres boxing 151G, 151D.

Bien entendu, les données mémorisées dans chaque enregistrement de la base de donnée pourraient comporter un nombre réduit d'éléments (par exemple seulement la largeur totale de la monture de lunettes) ou un nombre accru d'éléments (par exemple la forme exacte des cercles, le matériau des montures de lunettes, l'angle pantoscopique et l'angle de galbe des montures de lunettes, ...).

En variante, ces moyens d'acquisition pourraient ne comporter qu'un simple clavier (physique ou affiché sur l'écran tactile) permettant au vendeur :

- de saisir la largeur de la monture de lunettes 110 sélectionnée, qu'il aura au préalable mesurée à l'aide d'un réglet, et
- de positionner sur l'image acquise deux curseurs au niveau des 2 points entre lesquels il aura mesuré la largeur de la monture.

Encore en variante, ces moyens d'acquisition pourraient ne comporter qu'un simple étalon de dimensions connues, à rapporter sur la monture de lunettes sélectionnée (par clipsage, collage ou tout autre moyen) pour obtenir sur chaque image acquise par la caméra une référence dont les dimensions sont connues, permettant ainsi de mettre cette image à l'échelle.

Comme le montre la figure 4, l'unité de calcul 250 du kiosque à lunettes 200 est quant à elle logée à l'intérieur du pied 202 du châssis 200. Elle est conçue pour piloter les différents composants électroniques du kiosque 200 et pour traiter les images acquises par la caméra 210.

Cette unité de calcul 250 comprend à cet effet un processeur (CPU), une mémoire vive (RAM), une mémoire morte (ROM), des convertisseurs analogiques-numériques (A/D), et différentes interfaces d'entrée, de sortie et de communication.

Grâce à ses interfaces d'entrée, l'unité de calcul 250 est adaptée à recevoir les images acquises par la caméra 210, la hauteur de la coulisse 205 dans le pied 202 mesurée par lesdits moyens de détermination, et le numéro de code-barres de la monture de lunettes sélectionnée, lu par le lecteur de code-barres 290.

Dans sa mémoire vive, l'unité de calcul 250 mémorise ainsi en continu ces différentes données.

Grâce à un logiciel enregistré dans sa mémoire morte, l'unité de calcul 250 est adaptée à mettre en oeuvre l'ensemble du procédé qui sera décrit dans la suite de cet exposé. Elle est ainsi par exemple adaptée à générer des signaux de pilotage des moyens d'actionnement de la bascule 205 pour positionner cette dernière à la hauteur souhaitée, et des signaux de communication comportant les données de commande des lentilles ophtalmiques.

Grâce à ses interfaces de sortie, l'unité de calcul 250 est adaptée à transmettre ces signaux de sortie aux différents composants électroniques du kiosque 200, notamment à la caméra 210, aux sources de lumière 220, 230, 240, à l'écran tactile 270, à l'imprimante multifonction 280, aux cibles 310, 320, 330 et aux moyens d'actionnement de la coulisse 205.

Grâce à ses interfaces de communication, l'unité de calcul 250 est adaptée à transmettre les signaux de communication à un centre de fabrication de lentilles ophtalmiques.

Il est enfin prévu un interrupteur de mise sous tension du kiosque 200.

Préalablement à la venue du porteur 1, le vendeur met le kiosque 200 sous tension à l'aide de l'interrupteur prévu à cet effet.

Lors de cette mise sous tension, l'unité de pilotage commande l'alimentation électrique des néons 260, de manière à mettre en lumière les paires de lunettes qui sont à disposition sur les ailes 206, 207 du châssis 201 du kiosque 200.

Les trois sources de lumière 220, 230, 240 infrarouges restent quant à elles éteintes.

Puis, lorsqu'un individu se présente, après lecture d'un message sur l'écran l'y invitant, il choisit une paire de lunettes 100 parmi l'ensemble de celles qui sont en présentation sur les ailes 206, 207 du châssis 201 du kiosque 200. Dans l'exemple représenté sur les figures, la paire de lunettes sélectionnée est du type cerclé. Puis, comme l'y invite un message affiché sur l'écran, l'individu appelle le vendeur pour la suite du protocole.

Il est ensuite prévu une opération de détermination d'une dimension caractéristique de la monture de lunettes 110 sélectionnée.

Comme cela a été exposé supra, cette opération est notamment prévue pour permettre de mettre les images acquises à l'échelle.

Au cours de cette opération, le vendeur passe le code-barres de la monture de lunettes 110 sélectionnée devant le lecteur de code-barres 290. L'unité de calcul 250, grâce au numéro de code-barres, cherche alors dans la base de données l'enregistrement correspondant à cette monture de lunettes 110, puis elle récupère et mémorise dans sa mémoire morte les données suivantes : la largeur totale de la monture de lunettes 110, le rapport entre la hauteur et la largeur des cadres boxing 151G, 151D des cercles 111D, 111G de cette monture de lunettes 110, et l'écart entre ces deux cadres boxing 151G, 151D.

Bien entendu, comme cela a été exposé supra, cette opération de lecture de code-barres pourrait être remplacée par une opération plus simple consistant pour le vendeur à mesurer la largeur totale de la monture de lunettes à l'aide d'un réglet, à la saisir sur un clavier virtuel affiché sur l'écran tactile, et à positionner sur l'image acquise les deux curseurs au niveau des deux points entre lesquels il a mesuré la largeur de la monture (cette opération de positionnement des deux points pouvant en variante être réalisée automatiquement).

Il est alors prévu une opération d'acquisition des prescriptions du porteur 1, que ce dernier aura préalablement obtenues chez un optométriste.

Ces prescriptions sont généralement inscrites sur une feuille de prescription et comportent en particulier le type de lentilles (simples, bifocales, à variation progressive de puissances, teintées, ...) et le pouvoir de réfringence que devront présenter les lentilles pour corriger les déficiences de vision du porteur (c'est-à-dire leurs puissances optiques sphériques, cylindriques et prismatiques et leurs axes de cylindre). Elles peuvent bien sûr comporter d'autres informations telles que, dans le cas des lentilles bifocales ou à variation progressive de puissances, une addition.

Lors de cette opération, le vendeur recueille alors la feuille de prescription et la scanne à l'aide de l'imprimante multifonction 280, de manière que l'unité de calcul 250 puisse mémoriser l'image scannée de cette feuille de prescription dans sa mémoire morte.

Le vendeur peut également demander au porteur de choisir les traitements qu'il souhaite obtenir sur ses lentilles ophtalmiques (anti-reflets, hydrophobes, ...) et saisir ces informations dans des champs affichés à cet effet par l'unité de calcul 250 sur l'écran tactile 270.

Une fois ces informations acquises, l'unité de calcul 250 commande la mise sous tension de la première cible 310.

Elle commande également l'affichage sur l'écran tactile 270 :

- d'un message indiquant que les opérations de mesure peuvent commencer,
- des images acquises « en temps réel » par la caméra 210, et
- de deux flèches orientées à l'opposée l'une de l'autre, l'une vers le haut et l'autre vers le bas, pour piloter le déplacement de la coulisse 205 vers le haut ou vers le bas.

Le vendeur invite alors tout d'abord le porteur 1 à chausser ses lunettes et à les garder ainsi pendant l'ensemble des examens qui vont suivre.

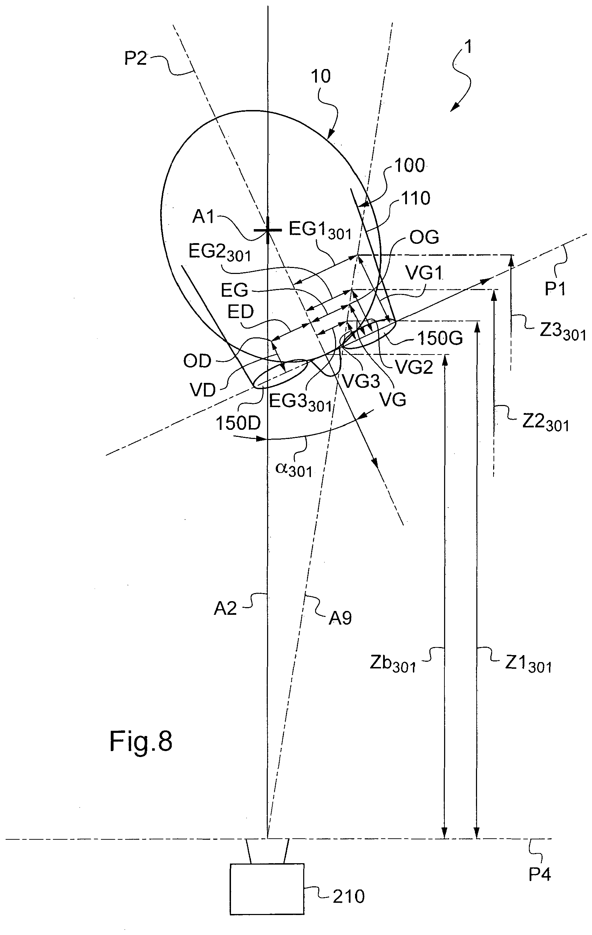

Comme cela sera exposé en détail dans la suite de cette description, ces examens permettront alors au kiosque 200 de déterminer les demi-écarts pupillaires EG, ED et les hauteurs pupillaires HG, HD du porteur 1, ainsi que des paramètres de personnalisation avancée tels que la distance VG, VD entre la monture de lunettes 110 et chaque oeil du porteur 1 (figure 8), le comportement en mobilité de son regard...

Il demande pour cela au porteur 1 de se placer face au kiosque 200, en position de mesure, et de regarder la première cible 310 en gardant la tête droite, de telle manière que son plan de Francfort P3 soit sensiblement horizontal et que son plan sagittal P2 soit sensiblement vertical.

Ici, le porteur 1 est invité à se placer debout, face à la coulisse 205 du châssis 201 du kiosque 200.

Le vendeur règle alors la position de la coulisse 205 à une hauteur adaptée à la taille du porteur 1. Il utilise à cet effet les flèches affichées sur l'écran tactile 270 pour commander la montée ou la descente de la coulisse 205 jusqu'à une hauteur telle que l'ensemble du visage du porteur 1 apparaisse sur les images acquises par la caméra 210 et affichées sur l'écran tactile 270.

Ainsi, dans cette position, le visage du porteur 1 se trouve tout entier dans le champ de la caméra 210.

Cette opération de positionnement de la caméra 210 à la hauteur de la tête 10 du porteur 1 pourrait bien entendu être réalisée autrement.

Elle pourrait ainsi être réalisée automatiquement par l'unité de calcul qui traiterait les images acquises par la caméra de manière à y repérer la monture de lunettes et qui piloterait en conséquence la bascule à une hauteur telle que la monture de lunettes se trouve au centre des images acquises par la caméra.

En variante, si le capteur d'images n'est pas monté mobile par rapport au sol, cette opération de positionnement pourra être réalisée en demandant au porteur de s'asseoir sur un tabouret dont la hauteur aura au préalable été réglée.

Une fois cette opération de positionnement du porteur 1 par rapport à la caméra 210 réalisée, le vendeur initie la prise de mesure en appuyant sur un bouton ad hoc affiché sur l'écran tactile 270.

L'unité de pilotage 250 commande alors l'allumage des trois sources de lumière 220, 230, 240 infrarouges, à une intensité nominale.

Bien entendu, l'unité de pilotage pourrait moduler cette intensité, en fonction de l'intensité de la lumière extérieure, pour éviter que cette dernière ne perturbe les mesures. L'utilisation d'une lumière infrarouge permet toutefois de limiter ces perturbations, si bien que l'intensité nominale est généralement suffisante.

L'utilisation de trois sources de lumière 220, 230, 240 distinctes assure alors la présence d'au moins un reflet sur chaque lentille de présentation 150G, 150D de la paire de lunettes 100, pour autant bien sûr que le porteur 1 ne détourne pas complètement la tête.

Dans la variante où le kiosque ne comporterait qu'une source de lumière mobile en hauteur par rapport à la caméra, l'opération de positionnement du porteur en face de la caméra serait suivie d'une opération automatique de positionnement de la source de lumière. Au cours de cette opération, l'unité de calcul ferait varier la hauteur de la source de lumière et traiterait les images acquises par la caméra pour immobiliser la source de lumière à une hauteur telle que les reflets-verres soient centrés sur les lentilles de présentation.

Quoi qu'il en soit, le vendeur demande alors au porteur 1 de tourner la tête 10 de droite à gauche et de gauche à droite, autour de l'axe longitudinal A1 (de manière à faire « non » de la tête).

Il lui indique que le mouvement doit être réalisé lentement (avec une période supérieure à la seconde), avec une amplitude réduite (d'environ 10 degrés).

En variante, on pourrait prévoir de monter la caméra et les sources de lumière mobiles de manière à ce qu'elles pivotent ensemble autour de l'axe longitudinal de la tête du porteur. Ainsi, le porteur n'aurait pas à faire de mouvement de tête.

Puis, lorsque le porteur 1 commence à tourner la tête de la manière demandée, le vendeur appuie sur un bouton de démarrage des mesures affiché sur l'écran tactile 270.

La caméra acquiert alors une pluralité d'images-infrarouges, sur lesquelles apparaissent la tête 10 du porteur 1 et sa paire de lunettes 100.

Les images acquises sont tout d'abord mémorisées dans la mémoire vive de l'unité de calcul 250. Certaines d'entre elles, jugées exploitables, sont alors sélectionnées par l'unité de calcul 250 et mémorisées dans sa mémoire morte. La sélection des images acquises est réalisée de la manière suivante.

Une image acquise peut présenter plusieurs reflets-verres (au maximum 6), chaque reflet-verre 160G, 160D, 161G, 161D est noté individuellement sur différents critères, notamment :

- de forme (largeur, hauteur, ratio largeur/hauteur), et
- d'intensité (luminance).

Chaque couple de reflets (générés par une même source de lumière et respectivement réfléchis par les deux lentilles de présentation) est par ailleurs noté selon d'autres critères, notamment :

- de distance entre les reflets,
- d'horizontalité par rapport à l'axe horizontal des cadres boxing, et
- de comparaison des surfaces des reflets.

Ici, la note attribuée à chaque critère n'est pas binaire.

On peut par exemple prévoir qu'elle varie continûment entre 0 et 1 de telle sorte que :

- elle soit égale à 1 si le critère est compris entre 90% et 110% de sa valeur nominale (prédéterminée et mémorisée dans la mémoire morte de l'unité de calcul 250),
- elle soit égale à 0 si le critère est supérieur à 120% ou inférieur à 70% de sa valeur nominale, et
- elle varie linéairement entre 70% et 90% et entre 110% et 120%.

Les critères s'appliquant aux reflets-verres peuvent également s'appliquer mutatis mutandis aux reflets cornéens 162G, 162D. Un critère supplémentaire s'appliquant aux reflets cornéens sera la distance séparant chaque reflet cornéen de l'axe remarquable A3.

Alors, une image a d'autant plus de chance d'être sélectionnée que le produit des notes attribuées aux différents critères précités est important.

On peut par exemple prévoir que les images sélectionnées sont celles pour lesquelles le produit des notes est supérieur à un seuil prédéterminé.

En variante, on peut prévoir que les images sélectionnées sont celles pour lesquelles les produits des notes sont les plus élevés.

On peut par ailleurs prévoir qu'une image est automatiquement rejetée si l'une des notes qui lui a été attribuée est inférieure à un autre seuil prédéterminé.

Ainsi, si sur une image acquise, le porteur 1 a tourné la tête d'un angle de lacet $\alpha$ trop important et que les reflets-verres sont sortis du contour des lentilles de présentation 150G, 150D, cette image est automatiquement rejetée.

Par ailleurs, si sur une image acquise, le porteur 1 a incliné la tête d'un angle de roulis β important et que les reflets-verres générés par une même source de lumière son décalés en hauteur sur les deux lentilles de présentation, cette image pourra être rejetée si ce décalage est vraiment très important, ou sélectionnée si les notes attribuées aux autres critères sont élevées.

L'unité de calcul 250 obtient ainsi un nombre restreint d'images-infrarouges exploitables pour la suite du procédé.

Pour la clarté de cet exposé, on considérera alors que l'unité de calcul 250 n'a sélectionné que trois images-infrarouges 301, 302, 303 représentées sur les figures 5 à 7.

Préférentiellement, l'unité de calcul arrêtera l'acquisition d'images lorsqu'elle aura sélectionnée au moins 10 images différentes.

L'unité de calcul 250 commande ensuite le basculement du filtre infrarouge de la caméra 210 pour mémoriser une image-visible du visage du porteur 1.

Elle commande simultanément l'extinction des sources de lumières 220, 230, 240 infrarouges, et l'affichage d'un message sur l'écran tactile 270 indiquant au porteur qu'il peut arrêter de tourner la tête.

Ce message est donc affiché après que l'unité de calcul 250 a sélectionné un nombre prédéterminé d'images, ici égal à 10.

En variante, on pourrait prévoir d'afficher ce message après une durée prédéterminée, par exemple égale à 10 secondes, à l'issue de laquelle on estime pouvoir obtenir un nombre suffisant d'images exploitables. Les images pourront alors être sélectionnées dans un second temps, après que l'ensemble des images ont été acquises.

Une fois les images 301, 302, 303 sélectionnées, l'unité de calcul 250 procède au calcul de l'angle de lacet α de la tête 10 du porteur 1 sur chacune de ces images.

Le calcul de cet angle de lacet α est opéré en localisant sur chaque image 301, 302, 303 les reflets-verres 160D, 160G, 161D, 161G par rapport au plan sagittal P2 de la tête du porteur 1.

Ici, cet angle de lacet α est plus précisément déterminé en localisant les reflets-verres 160D, 160G, 161D, 161G par rapport à l'axe remarquable A3 de la monture de lunettes (dont la position correspond en effet sensiblement à l'intersection du plan sagittal P2 avec le plan moyen P1 de la monture de lunettes 110).

La position de l'axe remarquable A3 de la monture de lunettes 110 est obtenu en 3 étapes consistant à :

- repérer sur chaque image 301, 302, 303 la monture de lunettes 110, et notamment les cercles 111D, 111G de cette monture de lunettes,
- positionner sur chaque image 301, 302, 303 les cadres boxing 151G, 151D, et
- déterminer la position de l'axe passant entre ces deux cadres boxing, qui correspond en pratique à l'axe remarquable A3.

L'utilisation d'une lumière infrarouge permet ici de faciliter le repérage de la monture de lunettes 110, dont le contour se détache bien de celui du visage du porteur 1.

Alors, l'angle de lacet α de la tête10 du porteur 1 sur chaque image 301, 302, 303 est calculé en fonction des distances séparant les reflets-verres 160D, 160G, 161D, 161G de l'axe remarquable A3.

Typiquement, comme le montrent bien les figures 5 à 7, l'unité de calcul 250 détermine sur chaque image 301, 302, 303 les distances $XD_{301}$, $XG_{301}$, $XD_{302}$, $XG_{302}$, $XD_{303}$, $XG_{303}$ séparant l'axe remarquable A3 des centres des deux reflets-verres 160G, 160D les plus gros (les reflets-verres générés par les sources secondaires n'étant exploités que si les reflets-verres générés par la source principale n'apparaissent par sur les images acquises).

L'angle de lacet $\alpha_i$ de la tête 10 du porteur 1 sur chaque image 301, 302, 303 est alors déterminé de la manière suivante :

$$\alpha_i = k. \ (XG_i - XD_i) \ / \ (XG_i + XD_i),$$

avec i allant de 301 à 303.

Le coefficient k est alors une constante relative au galbe de la monture de lunettes.

Ce coefficient k pourra être formé par une constante prédéterminée et invariable, mémorisée dans la mémoire morte de l'unité de calcul 250. Elle sera donc toujours la même quelle que soit la monture de lunettes sélectionnée par le porteur. Ce coefficient k pourra alors être déterminé expérimentalement sur la base d'un panel représentatif de montures de lunettes, et ainsi choisi environ égal à 40°.

En variante, ce coefficient k pourra être formé par une constante prédéterminée associée à la monture de lunettes sélectionnée par le client. Dans cette variante, ce coefficient k sera alors déterminé expérimentalement pour chaque référence de monture de lunettes, puis mémorisé dans la base de données de manière à être accessible lors du calcul de l'angle de lacet $\alpha_i$ de la tête 10 du porteur 1.

La détermination de cet angle de lacet pourrait être réalisée autrement.

A titre d'exemple, on pourrait prévoir d'équiper la monture de lunettes d'un système de repérage comportant des motifs géométriques facilement repérables et écartés d'une distance réelle connue, et de déterminer l'angle de lacet en fonction de la distance séparant ces motifs géométriques sur l'image acquise (préalablement mise à l'échelle 1 : 1). Un tel procédé est présenté en détail dans le document WO 2008/132356.

A ce stade, l'unité de calcul 250 a en mémoire les angles de lacet $\alpha_{301}$, $\alpha_{302}$, $\alpha_{303}$ de la tête 10 du porteur 1 sur chaque image 301, 302, 303 sélectionnée.

Elle procède alors à la détermination des demi-écarts pupillaires EG, ED du porteur 1, en fonction des positions des reflets cornéens 162G, 162D du porteur 1 sur au moins une des images 301, 302, 303 sélectionnées.

La méthode selon l'invention consiste à considérer toutes les images 301, 302, 303 sélectionnées, puis, par une méthode statistique, à évaluer les demi-écarts pupillaires EG, ED du porteur 1.

Cette méthode statistique permet non seulement d'évaluer les demi-écarts pupillaires EG, ED du porteur 1, mais également d'évaluer les distances oeil-lunettes VG, VD séparant le plan moyen P1 de la monture de lunettes 110 et les centres de rotation OG, OD des yeux 20G, 20D du porteur 1.

Cette méthode statistique est alors mise en oeuvre sur l'un des deux yeux 20G, 20D du porteur 1.

Ici, comme le montre la figure 8, elle sera mise en oeuvre sur l'œil gauche 20G du porteur 1 afin de déterminer son demi-écart pupillaire gauche EG et sa distance oeil-lunettes gauche VG.

Globalement la méthode statistique part du constat que, grâce au reflet cornéen gauche 162G apparaissant sur chaque image 301, 302, 303, on sait sur quel axe A9 (voir figure 8) se trouve le centre de rotation OG de l'œil gauche 20G du porteur 1, et qu'il reste donc seulement à déterminer la position du centre de rotation OG sur cet axe A9.

Cette méthode statistique va alors consister :

- à simuler, sur chaque image 301, 302, 303, une pluralité de distances oeil-lunettes VG1, VG2, VG3 (ici au nombre de 3 pour la clarté de l'exposé et des dessins),
- à déterminer le demi-écart pupillaire gauche correspondant à chacune de ces distances simulées (appelé « demi-écart pupillaire gauche simulé $EG1_{301}$, $EG2_{301}$, $EG3_{301}$, $EG1_{302}$, $EG2_{302}$, $EG3_{302}$, $EG1_{303}$, $EG2_{303}$, $EG3_{303}$»), puis, en fonction de l'ensemble de ces demi-écarts pupillaires gauches simulés,
- à déterminer laquelle des distances oeil-lunettes simulées VG1, VG2, VG3 est la plus proche de la distance oeil-lunette VG réelle du porteur 1.

Plus précisément, l'unité de calcul 250 considère ici trois distances oeil-lunettes simulées VG1, VG2, VG3 prédéterminées et mémorisées dans sa mémoire morte. En variante et de manière préférentielle, elle considérera plutôt un nombre de distances oeil-lunettes simulées supérieur à 10.

Alors, pour chacune de ces distances oeil lunettes simulées, et pour chacune des images acquises (indice i, variant dans cet exemple de 301 à 303), l'unité de calcul 250 calcule de manière itérative (indice d'itération k variant de 1 à n) :

$$Z1_{i,k} = Zb_i + VG1.\cos(\alpha_i) + EG1_{i,k-1}.\sin(\alpha_i),$$

$$Z2_{i,k} = Zb_i + VG2.\cos(\alpha_i) + EG2_{i,k-1}.\sin(\alpha_i),$$

$$Z3_{i,k} = Zb_i + VG3.\cos(\alpha_i) + EG3_{i,k-1}.\sin(\alpha_i),$$

avec :

- $EG1_{i,0}$, $EG2_{i,0}$, $EG3_{i,0}$ fixé arbitrairement à une valeur prédéterminée, ici choisie égale à 32,5 mm ;
- $Zb_i$ la distance séparant l'axe remarquable A3 et le plan général P4 de l'objectif de la caméra 210 sur chaque image (et qui est facilement déterminable, compte tenu de la largeur de la monture sur l'image considérée par rapport à sa largeur réelle, et de l'angle de lacet $\alpha_i$ de la tête du porteur) ;
- $Z1_{i,k}$, $Z2_{i,k}$, $Z3_{i,k}$ les distances séparant la position simulée du centre de rotation OG de l'œil gauche 20G du porteur 1 et le plan général P4 de l'objectif de la caméra 210.

Si on note :

- $x_{OGi}$ et $y_{OGi}$ : les coordonnées, exprimées en pixel, du centre de rotation OG dans l'image i, par rapport au centre de cette image,

- X,Y,Z : les coordonnées, exprimées en mm, du centre de rotation OG dans le repère de la caméra, et
- K, la taille d'un pixel, exprimée en mm, pour un objet situé à un mètre du plan de la caméra.

Alors, on peut écrire les équations suivantes :

$$X1_{i,k} = Z1_{i,k} \cdot K \cdot x_{OGi}$$

$$Y1_{i,k} = Z1_{i,k} \cdot K \cdot y_{OGi}$$

$$Z1_{i,k} = Z1_{i,k}$$

$$X2_{i,k} = Z2_{i,k} \cdot K \cdot x_{OGi}$$

$$Y2_{i,k} = Z2_{i,k} \cdot K \cdot y_{OGi}$$

$$Z2_{i,k} = Z2_{i,k}$$

$$X3_{i,k} = Z3_{i,k} \cdot K \cdot x_{OGi}$$

$$Y3_{i,k} = Z3_{i,k} \cdot K \cdot y_{OGi}$$

$$Z3_{i,k} = Z3_{i,k}$$

Pour l'image i, le plan sagittal, noté $P2_i$ peut être, par exemple, défini comme le plan qui inclut les trois points suivants :

- $A_i$ : un point situé sur l'axe remarquable A3, correspondant au centre du segment reliant les deux coins supérieurs les plus proches des deux cadres boxing 151G, 151D,
- $B_i$ : un point situé sur l'axe remarquable A3, correspondant au centre du segment reliant les deux coins inférieurs les plus proches des deux cadres boxing 151G, 151D,
- Ci : un point situé sur un axe qui passe par le point $A_i$, qui est perpendiculaire au plan moyen P1 de la monture de lunettes 110, et dont les coordonnées dans le repère de la monture sont telles que le vecteur $A_iC_i$ a pour coordonnées $(x_c, y_c, z_c)$ avec $x_c = 0$ et $y_c = 0$.

Si on pose :

- $(x_{Ai}, y_{Ai})$ les coordonnées du point $A_i$ dans l'image i,
- $X_{Ai}, Y_{Ai}, Z_{Ai}$, les coordonnées du point $A_i$ dans le repère de la caméra,
- $(x_{Bi}, y_{Bi})$ les coordonnées du point $A_i$ dans l'image i,
- $X_{Bi}, Y_{Bi}, Z_{Bi}$ les coordonnées du point $B_i$ dans le repère de la caméra,
- $X_{Ci}, Y_{Ci}, Z_{Ci}$ les coordonnées du point Ci dans le repère de la caméra, et
- $M_i$, la matrice de rotation tridimensionnelle décrivant la posture de la monture dans l'espace de la caméra (construite à partir des angles $\alpha$, $\beta$ et $\delta$).

Alors, comme la distance de l'axe remarquable A3 au plan de la caméra P4 est connue, $Z_{Ai}$ et $Z_{Bi}$ sont connus et on peut écrire les équations suivantes :

$$X_{Ai} = Z_{Ai} \cdot K \cdot x_{Ai}$$

$$Y_{Ai} = Y_{Ai} \cdot K \cdot y_{Ai}$$

$$X_{Bi} = Z_{Bi} \cdot K \cdot x_{Bi}$$

$$Y_{Bi} = Y_{Bi} \cdot K \cdot y_{Bi}$$

Et, après inversion de la matrice de rotation M :

$$(X_{Ci} - X_{Ai}) = M^{-1}(0,0) \cdot x_c + M^{-1}(0,1) \cdot y_c + M^{-1}(0,2) \cdot z_c$$

$$(Y_{Ci} - Y_{Ai}) = M^{-1}(1,0) \cdot x_c + M^{-1}(1,1) \cdot y_c + M^{-1}(1,2) \cdot z_c$$

$$(Z_{Ci} - Z_{Ai}) = M^{-1}(2,0) \cdot x_c + M^{-1}(2,1) \cdot y_c + M^{-1}(2,2) \cdot z_c$$

Comme, $x_c$ et $y_c$ sont égaux à zéro par construction, on en déduit que :

$$X_{Ci} = X_{Ai} + M^{-1}(0,2) \cdot z_c$$

$$Y_{Ci} = Y_{Ai} + M^{-1}(1,2) \cdot z_c$$

$$Z_{Ci} = Z_{Ai} + M^{-1}(2,2) \cdot z_c$$

Les coordonnées des 3 points du plan $P2_i$ étant connues, celui-ci est parfaitement défini, et il est donc possible de calculer selon la méthode classique la distance d'un point quelconque au plan $P2_i$.

On peut donc en particulier calculer $EG1_{i,k}$, $EG2_{i,k}$, $EG3_{i,k}$, avec

- $EG1_{i,k}$ : la distance séparant le centre de rotation OG du plan $P2_i$, en considérant la distance œil-lunettes VG1 ;
- $EG2_{i,k}$ : la distance séparant le centre de rotation OG du plan $P2_i$, en considérant la distance œil-lunettes VG2 ;
- $EG3_{i,k}$ : la distance séparant le centre de rotation OG du plan $P2_i$, en considérant la distance œil-lunettes VG3.

Ces valeurs peuvent alors être réinjectées dans les formules initiales pour continuer l'itération.

Le calcul itératif s'arrête soit après un nombre prédéterminé d'itérations (par exemple pour k = 3), soit lorsque pour deux itérations successives, les valeurs de $EG1_{i,k}$ et $EG1_{i,k+1}$, celles de $EG2_{i,k}$ et $EG2_{i,k+1}$ et celles de $EG3_{i,k}$ et $EG3_{i,k+1}$ sont proches (à savoir ici inférieures à 0,1 mm).

Les valeurs de $EG1_{i,k}$, $EG2_{i,k}$, $EG3_{i,k}$ obtenues sont alors considérées comme étant les demi-écarts pupillaires simulés $EG1_i$, $EG2_i$, $EG3_i$.

Une fois l'ensemble des demi-écarts pupillaires simulés $EG1_i$, $EG2_i$, $EG3_i$ calculés, l'unité de calcul 250 sélectionne, parmi les distances oeil-lunettes simulées VG1, VG2, VG3, celle qui est la plus proche de la valeur réelle VG.

Pour cela, l'unité de calcul 250 détermine, pour chaque distance oeil-lunettes simulée VG1, VG2, VG3, l'écart-type $\sigma_1$, $\sigma_2$, $\sigma_3$ des demi-écarts pupillaires simulés $EG1_i$, $EG2_i$, $EG3_i$ associés.

A titre d'exemple, l'écart-type $\sigma_1$ associé à la première distance oeil-lunettes simulée VG1 est égal à l'écart-type des demi-écarts pupillaires simulés $EG1_{303}$, $EG1_{302}$, $EG1_{303}$.

Alors, la distance oeil-lunettes simulée VG2 sélectionnée est celle pour laquelle l'écart-type $\sigma_2$ est le plus faible.

L'unité de calcul 250 considère alors que cette distance oeil-lunettes simulée VG2, une fois mise à l'échelle, est égale à la distance oeil-lunettes VG réelle du porteur 1. Elle mémorise donc sa valeur.

L'unité de calcul 250 calcule ensuite la moyenne des demi-écarts pupillaires simulés $EG2_{301}$, $EG2_{302}$, $EG2_{303}$

associés à cette valeur simulée sélectionnée VG2.

Elle considère ensuite que cette moyenne, une fois mise à l'échelle, est égale au demi-écart pupillaire gauche EG du porteur 1. Elle mémorise donc sa valeur.

La détermination de la distance oeil-lunettes droite VD et du demi-écart pupillaire droit ED du porteur 1 peut alors être réalisée de la même manière que pour les distances oeil-lunettes gauche VG et demi-écart pupillaire gauche EG du porteur 1.

Au cours d'une opération suivante, l'unité de calcul 250 procède au calcul des hauteurs pupillaires HG, HD du porteur 1 (voir figure 1).

Encore une fois, l'unité de calcul 250 peut opérer de diverses manières.

Ici, on considérera que le tangage de la tête 10 du porteur 1 a peu d'incidence sur la précision des hauteurs pupillaires HG, HD que l'on peut mesurer sur les images 301, 302, 303.

Par conséquent, pour déterminer ces hauteurs pupillaires, l'unité de calcul 250 sélectionne l'une quelconque des images 301, 302, 303 (par exemple celle pour laquelle l'angle de lacet $\alpha_{302}$ est le plus faible) puis détermine sur cette image 302 (figure 6) les distances $HG_{302}$, $HD_{302}$ séparant chacun des reflets cornéens 162G, 162D du bord inférieur du cadre boxing 151G, 151D correspondant.

Ces distances $HG_{302}$, $HD_{302}$, une fois mises à l'échelle, sont alors mémorisées par l'unité de calcul 250 comme étant les hauteurs pupillaires HG, HD.

En variante, l'unité de calcul pourra mesurer ces distances $HG_i$, $HD_i$ sur chacune des images 301, 302, 303, moyenner ces distances et les mettre à l'échelle afin d'obtenir les hauteurs pupillaires du porteur.

Selon une autre variante, on pourra procéder au calcul des hauteurs pupillaires en suivant une méthode homologue de celle utilisée pour déterminer les demi-écarts pupillaires du porteur. Cette méthode consistera par exemple à acquérir différentes images du porteur faisant « oui » de la tête, à déterminer les angles de tangage de la tête sur chacune de ces images, et à mesurer les hauteurs pupillaires sur l'image pour laquelle l'angle de tangage est le plus faible.

L'opération suivante consiste pour l'unité de calcul 250 à déterminer la propension du porteur 1 à détourner le regard en bougeant plutôt la tête 10 ou les yeux 20G, 20D.

Le vendeur explique alors au porteur 1 qu'il doit regarder la première cible supplémentaire 320, puis, dès que celle-ci s'éteint et que la seconde cible supplémentaire 330 s'illumine, qu'il doit détourner rapidement le regard vers cette seconde cible supplémentaire 330.

Dès que le porteur 1 est prêt, le vendeur initie une nouvelle prise de mesures en appuyant sur un bouton ad hoc affiché sur l'écran tactile 270.

L'unité de calcul 250 commande alors l'allumage de la première cible supplémentaire 320 seule, pendant environ 5 secondes, puis l'extinction de la première cible supplémentaire 320 et l'allumage simultané de la seconde cible supplémentaire 330. Au moment de l'extinction de la première cible supplémentaire 320, l'unité de calcul 250 commande l'acquisition et la mémorisation d'une pluralité d'images (par exemple 10) au cours d'une séquence d'environ 1 seconde.

Les images acquises sont alors traitées par l'unité de calcul 250 pour déterminer sur chacune d'entre elles l'angle de lacet $\alpha_i$ de la tête 10 du porteur 1.

Si, sur les 10 images acquises, cet angle varie peu et/ou lentement, l'unité de calcul 250 détermine que le porteur a une propension à détourner le regard en bougeant plutôt les yeux 20G, 20D.

Au contraire, si, sur les 10 images acquises, cet angle varie beaucoup et/ou rapidement, l'unité de calcul 250 détermine que le porteur a une propension à détourner le regard en bougeant plutôt la tête 10.

En variante, l'unité de calcul pourrait procéder autrement.

A titre d'exemple, elle pourrait procéder plus finement, en déterminant également sur chaque image acquise les distances $EG_i$, $ED_i$ séparant les reflets cornéens 162D, 162G de l'axe remarquable A3.

Elle pourrait alors comparer les vitesses et/ou amplitudes de variation de l'angle de lacet $\alpha_i$ avec les vitesses et/ou amplitudes de variation des distances $EG_i$, $ED_i$. Elle pourrait ainsi en déduire si le porteur a une propension à tourner plus rapidement les yeux ou la tête. Elle pourrait même en déduire un coefficient fin, quantifiant la propension du porteur à détourner le regard en bougeant plutôt la tête ou les yeux.

A l'issue de ces différentes opérations, l'unité de calcul 250 a acquis :

- une image scannée des prescriptions du porteur,
- la référence de la monture de lunettes sélectionnée par le porteur 1,
- les demi-écarts pupillaires EG, ED du porteur 1,
- les hauteurs pupillaires HG, HD du porteur 1,
- une image-visible du visage du porteur 1,
- les distances VG2, VD2 séparant les centres de rotation des yeux du porteur 1 et le plan moyen P1 de la monture de lunettes 110, et
- un coefficient quantifiant la propension du porteur 1 à détourner le regard en bougeant plutôt la tête 10 ou les yeux 20G, 20D.

Elle commande alors l'affichage de ces différentes informations sur l'écran tactile 270 pour vérification par le vendeur.

Après validation par le vendeur, l'unité de calcul 250 commande l'impression de ces informations sur une fiche récapitulative. Elle communique par ailleurs ces informations au centre de fabrication des lentilles ophtalmiques.

On pourra éventuellement prévoir que l'unité de calcul 250 communique en outre au centre de fabrication des lentilles ophtalmiques l'ensemble des images acquises.

Alors, le centre de traitement pourra éventuellement réaliser une opération de vérification de toutes les données calculées par l'unité de calcul 250.

Ces données pourront notamment être vérifiées lorsque le logiciel installé dans l'unité de calcul 250 n'aura pas été mis à jour.

Elles pourront également être vérifiées lorsque la monture sélectionnée sera de type « sans cercle » (ou « percée »). En effet, les bords des lentilles de présentation de ces montures sont généralement peu visibles sur les images acquises, ce qui peut générer des erreurs.

Cette opération de vérification pourra être mise en oeuvre automatiquement (notamment dans le cas où le logiciel n'est pas à jour), ou manuellement, par un technicien spécialisé qui pourra notamment vérifier que les pupilles et les bords des lentilles de présentation ont bien été repérés.

On pourra en particulier prévoir que le dispositif de mesure se présente, non pas sous la forme d'un kiosque logeant l'unité de calcul, le capteur d'images et les sources de lumière, mais plutôt sous une forme de taille réduite et portable. On pourra ainsi prévoir que l'unité de calcul soit formée par un ordinateur portable dans lequel sera installé un logiciel ad hoc, et dont la caméra (ou « web-cam ») fera office de caméra dans le domaine du visible. Dans cette variante, la caméra infrarouge et la source de lumière seront alors agencées sur un boîtier de petite taille, muni d'une pince de fixation à l'écran de l'ordinateur portable et d'un câble de branchement à l'ordinateur portable. Cette solution sera peu onéreuse et facilement transportable.

Selon une autre variante de réalisation de l'invention, on pourra prévoir que l'unité de calcul se contente d'enregistrer des images et de les communiquer au centre de fabrication de lentilles ophtalmiques, auquel cas ces images seront traitées dans ce centre de fabrication.

## Revendications

1. Procédé d'estimation d'une distance oeil-lunettes (VG), entre un plan caractéristique (P1) d'une paire de lunettes (100) et un point caractéristique (OG) d'un oeil d'un porteur (1) de ladite paire de lunettes (100), ledit plan caractéristique (P1) de la paire de lunettes (100) étant sensiblement parallèle au plan moyen des contours de lentilles (150G, 150D) de la paire de lunettes (100), à l'aide d'un dispositif de détermination (200) comprenant un capteur d'images (210), le procédé comportant des étapes :

   a) d'acquisition par le capteur d'images (210) d'au moins deux images (301, 302, 303) distinctes d'au moins une partie de la tête (10) du porteur (1), sur lesquelles la tête (10) du porteur (1) présente différentes positions angulaires ($\alpha$) par rapport au capteur d'images (210) autour d'un axe de pivotement (A1) qui est sensiblement parallèle audit plan caractéristique (P1) et qui est sensiblement transversal à l'axe optique (A2) du capteur d'images,
   b) de détermination par une unité de calcul (250), pour chaque image (301, 302, 303) acquise, d'un angle d'inclinaison ($\alpha_{301}$, $\alpha_{302}$, $\alpha_{303}$) de la tête (10) du porteur (1) par rapport au capteur d'images (210), mesuré entre l'axe optique (A2) du capteur d'images (210) et un plan (P2) attaché à la tête (10) du porteur (1) qui est sensiblement parallèle audit axe de pivotement (A1) et sensiblement orthogonal audit plan caractéristique (P1),

   **caractérisé en ce qu'**il comporte également des étapes :

   c) d'acquisition par ladite unité de calcul (250) d'au moins deux valeurs simulées (VG1, VG2, VG3) prédéterminées de la distance oeil-lunettes recherchée,
   d) de calcul trigonométrique de façon itérative par ladite unité de calcul (250), pour chaque image (301, 302, 303) acquise et avec chaque valeur simulée (VG1, VG2, VG3) acquise, d'une distance morphologique ($EG1_{301}$, $EG2_{301}$, $EG3_{301}$, $EG1_{302}$, $EG2_{302}$, $EG3_{302}$, $EG1_{303}$, $EG2_{303}$, $EG3_{303}$) entre le point caractéristique (OG) de l'œil (10) du porteur (1) et le plan (P2) attaché à la tête (10) du porteur (1), compte tenu dudit angle d'inclinaison ($\alpha_{301}$, $\alpha_{302}$, ($\alpha_{303}$), et
   e) de sélection par ladite unité de calcul (250), parmi les valeurs simulées (VG1, VG2, VG3) de la distance œil-lunettes, de la valeur la plus proche de la valeur réelle de la distance oeil-lunettes (VG) du porteur, en fonction des distances morphologique (EG1$_{301}$, EG2$_{301}$, EG3$_{301}$, EG1$_{302}$, EG2$_{302}$, EG3$_{302}$, EG1$_{303}$, EG2$_{303}$, EG3$_{303}$) calculées à l'étape d),

dans lequel à l'étape e) :

- on détermine, pour chaque valeur simulée (VG1, VG2, VG3), un taux de dispersion des distances morphologiques associées à cette valeur simulée (VG1, VG2, VG3), et
- la valeur simulée sélectionnée (VG2) est celle pour laquelle les distances morphologiques sont les moins dispersées.

2. Procédé d'estimation selon la revendication précédente, dans lequel, à l'étape a), ledit axe de pivotement (A1) est confondu avec l'axe longitudinal de la tête (10) du porteur (1), et à l'étape b), le plan (P2) attaché à la tête (10) du porteur (1) est parallèle au plan sagittal de la tête (10) du porteur (1) et ledit angle d'inclinaison ($\alpha_{301}$, $\alpha_{302}$, $\alpha_{303}$) est un angle de lacet.

3. Procédé d'estimation selon la revendication précédente, dans lequel le point caractéristique (OG) de l'oeil (10) du porteur (1) est le centre de rotation de l'un des deux yeux du porteur (1), le plan (P2) attaché à la tête (10) du porteur (1) est confondu avec le plan sagittal de la tête (10) du porteur (1), et chaque distance morphologique calculée à l'étape d) est un demi-écart pupillaire supposé du porteur (1) calculé en fonction de la position sur l'image (301, 302, 303) acquise de la position d'un reflet cornéen (162G) réfléchi par la cornée dudit oeil du porteur (1).

4. Procédé d'estimation selon l'une des revendications précédentes, dans lequel la position du plan (P2) attaché à la tête (10) du porteur (1) est obtenue en repérant la position de la paire de lunettes (100) sur chacune des images (301, 302, 303) acquises.

5. Procédé d'estimation selon l'une des revendications précédentes, dans lequel le taux de dispersion des distances morphologiques est un écart-type ($\sigma$).

6. Procédé d'estimation selon la revendication 4, comportant une étape supplémentaire d'estimation du demi-écart pupillaire (EG) du porteur (1), au cours de laquelle est calculée la moyenne des distances morphologiques (EG2$_{301}$, EG2$_{302}$, EG2$_{303}$) associées à la valeur simulée sélectionnée.

7. Procédé d'estimation selon l'une des revendications précédentes, dans lequel, le dispositif comportant une source de lumière (220), à l'étape b) ledit angle d'inclinaison ($\alpha_{301}$, $\alpha_{302}$, $\alpha_{303}$) est déterminé en fonction de la position, sur chaque image (301, 302, 303), des reflets (160D, 160G, 161D, 161G) générés par la source de lumière (220) et réfléchis par les deux lentilles (150G, 150D) de la paire de lunettes (100).

8. Procédé d'estimation selon la revendication précédente, dans lequel ladite source de lumière (220) est infrarouge et ledit capteur d'images (210) est adapté à acquérir des images (301, 302, 303) infrarouges.

9. Procédé d'estimation selon l'une des revendications précédentes, dans lequel ledit angle d'inclinaison ($\alpha_{301}$, $\alpha_{302}$, $\alpha_{303}$) est obtenu en installant sur la paire de lunettes (100) un élément de repérage ayant une géométrie connue, et en déterminant la position et la forme dudit élément de repérage sur chaque image (301, 302, 303) acquise.

10. Procédé d'estimation selon l'une des revendications précédentes, dans lequel on acquiert une dimension caractéristique de ladite paire de lunettes (100) et dans lequel ladite distance oeil-lunettes (VG) du porteur est mise à l'échelle en fonction de ladite dimension caractéristique acquise.

11. Dispositif (200) d'estimation d'une distance oeil-lunettes (VG), entre un plan caractéristique (P1) d'une paire de lunettes (100) et un point caractéristique (OG) d'une tête (10) d'un porteur (1) de ladite paire de lunettes (100), ledit plan caractéristique (P1) de la paire de lunettes (100) étant sensiblement parallèle au plan moyen des contours de lentilles (150G, 150D) de la paire de lunettes (100), ledit dispositif (200) comprenant :

- au moins une source de lumière (220) adaptée à éclairer la tête (10) du porteur (1),
- un capteur d'images (210) présentant un axe optique (A2) sensiblement dirigé vers la tête (10) du porteur (1) et adapté à acquérir au moins deux images (301, 302, 303) de la tête (10) du porteur (1) sur lesquelles apparaît la paire de lunettes (100) éclairée par la source de lumière (220) et sur lesquelles la tête (10) du porteur (1) présente des inclinaisons distinctes autour d'un axe de pivotement (A1) sensiblement parallèle audit plan caractéristique (P1) et transversal à l'axe optique (A2) du capteur d'images (210), et
- une unité de calcul (250) adaptée à traiter lesdites images (301, 302, 303),

**caractérisé en ce que** ladite unité de calcul (250) est adaptée à :

- déterminer, pour chaque image (301, 302, 303) acquise, un angle d'inclinaison ($\alpha_{301}$, $\alpha_{302}$, $\alpha_{303}$) de la tête (10) du porteur par rapport au capteur d'image (210), mesuré entre l'axe optique (A2) du capteur d'images (210) et un plan (P2) attaché à la tête (10) du porteur (1) qui est sensiblement parallèle audit axe de pivotement (A1) et sensiblement orthogonal audit plan caractéristique (P1),
- acquérir au moins deux valeurs simulées (VG1, VG2, VG3) prédéterminées de la distance oeil-lunettes recherchée,
- calculer de façon itérative, pour chaque image acquise (301, 302, 303) et avec chaque valeur simulée (VG1, VG2, VG3) acquise, une distance morphologique ($EG1_{301}$, $EG2_{301}$, $EG3_{301}$, $EG1_{302}$, $EG2_{302}$, $EG3_{302}$, $EG1_{303}$, $EG2_{303}$, $EG3_{303}$) entre le point caractéristique (OG) de la tête (10) du porteur (1) et le plan (P2) attaché à la tête (10) du porteur (1), compte tenu dudit angle d'inclinaison ($\alpha_{301}$, $\alpha_{302}$, $\alpha_{303}$), et
- sélectionner, parmi les valeurs simulées (VG1, VG2, VG3) de la distance œil-lunettes, la valeur la plus proche de la distance réelle œil-lunettes (VG) du porteur, en fonction des distances morphologique ($EG1_{301}$, $EG2_{301}$, $EG3_{301}$, $EG1_{302}$, $EG2_{302}$, $EG3_{302}$, $EG1_{303}$, $EG2_{303}$, $EG3_{303}$) calculées, ladite sélection étant opérée en déterminant, pour chaque valeur simulée (VG1, VG2, VG3), un taux de dispersion des distances morphologiques associées à cette valeur simulée (VG1, VG2, VG3), la valeur simulée sélectionnée (VG2) étant celle pour laquelle les distances morphologiques sont les moins dispersées.

**Patentansprüche**

1. Verfahren zum Schätzen eines Auge-Brille-Abstands (VG) zwischen einer charakteristische Ebene (P1) einer Brille (100) und einem charakteristische Punkt (OG) eines Auges eines Trägers (1) der Brille (100), wobei die charakteristische Ebene (P1) der Brille (100) im Wesentlichen parallel zur Mittelebene der Linsenkonturen (150G, 150D) der Brille (100) ist, mit Hilfe einer Bestimmungsvorrichtung (200), die einen Bildsensor (210) enthält, wobei das Verfahren Schritte enthält:

a) der Erfassung durch den Bildsensor (210) von mindestens zwei unterschiedlichen Bildern (301, 302, 303) mindestens eines Teils des Kopfes (10) des Trägers (1), auf denen der Kopf (10) des Trägers (1) verschiedene Winkelpositionen ($\alpha$) bezüglich des Bildsensors (210) um eine Schwenkachse (A1) aufweist, die im Wesentlichen parallel zur charakteristischen Ebene (P1) und im Wesentlichen quer zur optischen Achse (A2) des Bildsensors ist,
b) der Bestimmung durch eine Recheneinheit (250), für jedes erfasste Bild (301, 302, 303), eines Neigungswinkels ($\alpha_{301}$, $\alpha_{302}$, $\alpha_{303}$) des Kopfes (10) des Trägers (1) bezüglich des Bildsensors (210), gemessen zwischen der optischen Achse (A2) des Bildsensors (210) und einer mit dem Kopf (10) des Trägers (1) verbundenen Ebene (P2), die im Wesentlichen parallel zur Schwenkachse (A1) und im Wesentlichen orthogonal zur charakteristischen Ebene (P1) ist,

**dadurch gekennzeichnet, dass** es ebenfalls Schritte aufweist:

c) der Erfassung durch die Recheneinheit (250) von mindestens zwei vorbestimmten simulierten Werten (VG1, VG2, VG3) des gesuchten Auge-Brille-Abstands,
d) der iterativen trigonometrischen Berechnung durch die Recheneinheit (250), für jedes erfasste Bild (301, 302, 303) und mit jedem erfassten simulierten Wert (VG1, VG2, VG3), eines morphologischen Abstands ($EG1_{301}$, $EG2_{301}$, $EG3_{301}$, $EG1_{302}$, $EG2_{302}$, $EG3_{302}$, $EG1_{303}$, $EG2_{303}$, $EG3_{303}$) zwischen dem charakteristischen Punkt (OG) des Auges (10) des Trägers (1) und der mit dem Kopf (10) des Trägers (1) verbundenen Ebene (P2), unter Berücksichtigung des Neigungswinkels ($\alpha_{301}$, $\alpha_{302}$, $\alpha_{303}$), und
e) der Auswahl durch die Recheneinheit (250), unter den simulierten Werten (VG1, VG2, VG3) des Auge-Brille-Abstands, des dem realen Wert am nächsten liegenden Werts des Auge-Brille-Abstands (VG) des Trägers, abhängig von den im Schritt d) berechneten morphologischen Abständen ($EG1_{301}$, $EG2_{301}$, $EG3_{301}$, $EG1_{302}$, $EG2_{302}$, $EG3_{302}$, $EG1_{303}$, $EG2_{303}$, $EG3_{303}$),

wobei im Schritt e) :

- für jeden simulierten Wert (VG1, VG2, VG3) ein Streuungsgrad der morphologischen Abstände bestimmt wird, die diesem simulierten Wert (VG1, VG2, VG3) zugeordnet sind, und
- der ausgewählte simulierte Wert (VG2) derjenige ist, für den die morphologischen Abstände die am wenigsten gestreuten sind.

2. Schätzverfahren nach dem vorhergehenden Anspruch, wobei im Schritt a) die Schwenkachse (A1) mit der Längsachse des Kopfes (10) des Trägers (1) zusammenfällt, und im Schritt b) die mit dem Kopf (10) des Trägers (1) verbundene Ebene (P2) parallel zur Sagittalebene des Kopfes (10) des Trägers (1) ist, und der Neigungswinkel ($\alpha_{301}$, $\alpha_{302}$, $\alpha_{303}$) ein Gierwinkel ist.

3. Schätzverfahren nach dem vorhergehenden Anspruch, wobei der charakteristische Punkt (OG) des Auges (10) des Trägers (1) das Drehzentrum eines der zwei Augen des Trägers (1) ist, die mit dem Kopf (10) des Trägers (1) verbundene Ebene (P2) mit der Sagittalebene des Kopfes (10) des Trägers (1) zusammenfällt, und jeder im Schritt d) berechnete morphologische Abstand ein angenommener halber Pupillenabstand des Trägers (1) ist, berechnet abhängig von der Position auf dem erfassten Bild (301, 302, 303) der Position eines von der Hornhaut des Auges des Trägers (1) reflektierten Hornhautreflexes (162G).

4. Schätzverfahren nach einem der vorhergehenden Ansprüche, wobei die Position der mit dem Kopf (10) des Trägers (1) verbundenen Ebene (P2) durch Markieren der Position der Brille (100) auf jedem der erfassten Bilder (301, 302, 303) erhalten wird.

5. Schätzverfahren nach einem der vorhergehenden Ansprüche, wobei der Streuungsgrad der morphologischen Abstände eine Standardabweichung ($\sigma$) ist.

6. Schätzverfahren nach Anspruch 4, das einen zusätzlichen Schritt der Schätzung des halben Pupillenabstands (EG) des Trägers (1) aufweist, während dessen der Mittelwert der morphologischen Abstände ($EG2_{301}$, $EG2_{302}$, $EG2_{303}$,) berechnet wird, die dem ausgewählten simulierten Wert zugeordnet sind.

7. Schätzverfahren nach einem der vorhergehenden Ansprüche, wobei, da die Vorrichtung eine Lichtquelle (220) aufweist, im Schritt b) der Neigungswinkel ($\alpha_{301}$, $\alpha_{302}$, $\alpha_{303}$) abhängig von der Position, auf jedem Bild (301, 302, 303), der von der Lichtquelle (220) erzeugten und von den zwei Linsen (150G, 150D) der Brille (100) reflektierten Reflexe (160D, 160G, 161D, 161G) bestimmt wird.

8. Schätzverfahren nach dem vorhergehenden Anspruch, wobei die Lichtquelle (220) infrarot und der Bildsensor (210) geeignet ist, Infrarotbilder (301, 302, 303) zu erfassen.

9. Schätzverfahren nach einem der vorhergehenden Ansprüche, wobei der Neigungswinkel ($\alpha_{301}$, $\alpha_{302}$, $\alpha_{303}$) durch Anbringen auf der Brille (100) eines Markierungselements mit einer bekannten Geometrie und durch Bestimmen der Position und der Form des Markierungselements auf jedem erfassten Bild (301, 302, 303) erhalten wird.

10. Schätzverfahren nach einem der vorhergehenden Ansprüche, wobei eine charakteristische Abmessung der Brille (100) erfasst wird, und wobei der Auge-Brille-Abstand (VG) des Trägers abhängig von der erfassten charakteristischen Abmessung normiert wird.

11. Vorrichtung (200) zur Schätzung eines Auge-Brille-Abstands (VG) zwischen einer charakteristischen Ebene (P1) einer Brille (100) und einem charakteristischen Punkt (OG) eines Kopfes (10) eines Trägers (1) der Brille (100), wobei die charakteristische Ebene (P1) der Brille (100) im Wesentlichen parallel zur Mittelebene der Linsenkonturen (150G, 150D) der Brille (100) ist, wobei die Vorrichtung (200) enthält:

- mindestens eine Lichtquelle (220), die geeignet ist, den Kopf (10) des Trägers (1) zu beleuchten,
- einen Bildsensor (210), der eine optische Achse (A2) im Wesentlichen zum Kopf (10) des Trägers (1) gerichtet hat und geeignet ist, mindestens zwei Bilder (301, 302, 303) des Kopfes (10) des Trägers (1) zu erfassen, auf denen die von der Lichtquelle (220) beleuchtete Brille (100) erscheint, und auf denen der Kopf (10) des Trägers (1) unterschiedliche Neigungen um eine Schwenkachse (A1) im Wesentlichen parallel zur charakteristischen Ebene (P1) und quer zur optischen Achse (A2) des Bildsensors (210) aufweist, und
- eine Recheneinheit (250), die geeignet ist, die Bilder (301, 302, 303) zu verarbeiten,

**dadurch gekennzeichnet, dass** die Recheneinheit (250) geeignet ist:

- für jedes erfasste Bild (301, 302, 303) einen Neigungswinkel ($\alpha_{301}$, $\alpha_{302}$, $\alpha_{303}$) des Kopfes (10) des Trägers bezüglich des Bildsensors (210) zu bestimmen, gemessen zwischen der optischen Achse (A2) des Bildsensors (210) und einer mit dem Kopf (10) des Trägers (1) verbundenen Ebene (P2), die im Wesentlichen parallel zur Schwenkachse (A1) und im Wesentlichen orthogonal zur charakteristische Ebene (P1) ist,

- mindestens zwei vorbestimmte simulierte Werte (VG1, VG2, VG3) des gesuchten Auge-Brille-Abstands zu erfassen,

- für jedes erfasste Bild (301, 302, 303) und mit jedem erfassten simulierten Wert (VG1, VG2, VG3) einen morphologischen Abstand ($EG1_{301}$, $EG2_{301}$, $EG3_{301}$, $EG1_{302}$, $EG2_{302}$, $EG3_{302}$, $EG1_{303}$, $EG2_{303}$, $EG3_{303}$) zwischen dem charakteristischen Punkt (OG) des Kopfes (10) des Trägers (1) und der mit dem Kopf (10) des Trägers (1) verbundenen Ebene (P2) unter Berücksichtigung des Neigungswinkels ($\alpha_{301}$, $\alpha_{302}$, $\alpha_{303}$) iterativ zu berechnen, und

- unter den simulierten Werten (VG1, VG2, VG3) des Auge-Brille-Abstands den dem realen Auge-Brille-Abstand (VG) des Trägers am nächsten liegenden Wert abhängig von den berechneten morphologischen Abständen ($EG1_{301}$, $EG2_{301}$, $EG3_{301}$, $EG1_{302}$, $EG2_{302}$, $EG3_{302}$, $EG1_{303}$, $EG2_{303}$, $EG3_{303}$) auszuwählen, wobei die Auswahl durchgeführt wird, indem für jeden simulierten Wert (VG1, VG2, VG3) ein Streuungsgrad der diesem simulierten Wert (VG1, VG2, VG3) zugeordneten morphologischen Abstände bestimmt wird, wobei der ausgewählte simulierte Wert (VG2) derjenige ist, für den die morphologischen Abstände die am wenigsten gestreuten sind.

## Claims

1. Method for estimating an eye-to-glasses distance (VG), between a characteristic plane (P1) of a pair of glasses (100) and a characteristic point (OG) of an eye of a wearer (1) of said pair of glasses (100), said characteristic plane (P1) of the pair of glasses (100) being substantially parallel to the average plane of the contours of lenses (150G, 150D) of the pair of glasses (100), using a determination device (200) comprising an image sensor (210), the method comprising the following steps:

   a) the image sensor (210) acquiring at least two separate images (301, 302, 303) of at least part of the head (10) of the wearer (1), in which images the head (10) of the wearer (1) has different angular positions ($\alpha$) with respect to the image sensor (210) about a pivoting axis (A1) that is substantially parallel to said characteristic plane (P1) and that is substantially transverse to the optical axis (A2) of the image sensor,

   b) a computing unit (250) determining, for each acquired image (301, 302, 303), an angle of inclination ($\alpha_{301}$, $\alpha_{302}$, $\alpha_{303}$) of the head (10) of the wearer (1) with respect to the image sensor (210), measured between the optical axis (A2) of the image sensor (210) and a plane (P2) attached to the head (10) of the wearer (1) that is substantially parallel to said pivoting axis (A1) and substantially orthogonal to said characteristic plane (P1),

   **characterized in that** it also comprises the following steps:

   c) said computing unit (250) acquiring at least two predetermined simulated values (VG1, VG2, VG3) of the sought eye-to-glasses distance,

   d) said computing unit (250) iteratively trigonometrically computing, for each acquired image (301, 302, 303) and with each acquired simulated value (VG1, VG2, VG3), a morphological distance ($EG1_{301}$, $EG2_{301}$, $EG3_{301}$, $EG1_{302}$, $EG2_{302}$, $EG3_{302}$, $EG1_{303}$, $EG2_{303}$, $EG3_{303}$) between the characteristic point (OG) of the eye (10) of the wearer (1) and the plane (P2) attached to the head (10) of the wearer (1), taking into account said angle of inclination ($\alpha_{301}$, $\alpha_{302}$, $\alpha_{303}$), and

   e) said computing unit (250) selecting, from among the simulated values (VG1, VG2, VG3) of the eye-to-glasses distance, the value closest to the real value of the eye-to-glasses distance (VG) of the wearer, on the basis of the morphological distances ($EG1_{301}$, $EG2_{301}$, $EG3_{301}$, $EG1_{302}$, $EG2_{302}$, $EG3_{302}$, $EG1_{303}$, $EG2_{303}$, $EG3_{303}$) computed in step d),

   wherein, in step e):

   - for each simulated value (VG1, VG2, VG3), a dispersion rate of the morphological distances associated with this simulated value (VG1, VG2, VG3) is determined, and
   - the selected simulated value (VG2) is the one for which the morphological distances are the least dispersed.

2. Estimation method according to the preceding claim, wherein, in step a), said pivoting axis (A1) is coincident with the longitudinal axis of the head (10) of the wearer (1), and, in step b), the plane (P2) attached to the head (10) of the wearer (1) is parallel to the sagittal plane of the head (10) of the wearer (1), and said angle of inclination ($\alpha_{301}$, $\alpha_{302}$, $\alpha_{303}$) is a yaw angle.

3. Estimation method according to the preceding claim, wherein the characteristic point (OG) of the eye (10) of the wearer (1) is the centre of rotation of one of the two eyes of the wearer (1), the plane (P2) attached to the head (10) of the wearer (1) is coincident with the sagittal plane of the head (10) of the wearer (1), and each morphological distance computed in step d) is an assumed half pupil distance of the wearer (1) computed on the basis of the position, in the acquired image (301, 302, 303), of the position of a corneal reflection (162G) reflected by the cornea of said eye of the wearer (1).

4. Estimation method according to one of the preceding claims, wherein the position of the plane (P2) attached to the head (10) of the wearer (1) is obtained by locating the position of the pair of glasses (100) in each of the acquired images (301, 302, 303).

5. Estimation method according to one of the preceding claims, wherein the dispersion rate of the morphological distances is a standard deviation ($\sigma$).

6. Estimation method according to Claim 4, comprising an additional step of estimating the half pupil distance (EG) of the wearer (1), in which the average of the morphological distances ($EG2_{301}$, $EG2_{302}$, $EG2_{303}$) associated with the selected simulated value is computed.

7. Estimation method according to one of the preceding claims, wherein, the device comprising a light source (220), in step b), said angle of inclination ($\alpha_{301}$, $\alpha_{302}$, $\alpha_{303}$) is determined on the basis of the position, in each image (301, 302, 303), of the reflections (160D, 160G, 161D, 161G) generated by the light source (220) and reflected by the two lenses (150G, 150D) of the pair of glasses (100).

8. Estimation method according to the preceding claim, wherein said light source (220) is infrared and said image sensor (210) is designed to acquire infrared images (301, 302, 303).

9. Estimation method according to one of the preceding claims, wherein said angle of inclination ($\alpha_{301}$, $\alpha_{302}$, $\alpha_{303}$) is obtained by installing a location element having a known geometry on the pair of glasses (100), and by determining the position and the shape of said location element in each acquired image (301, 302, 303).

10. Estimation method according to one of the preceding claims, wherein a characteristic dimension of said pair of glasses (100) is acquired and wherein said eye-to-glasses distance (VG) of the wearer is scaled on the basis of said acquired characteristic dimension.

11. Device (200) for estimating an eye-to-glasses distance (VG), between a characteristic plane (P1) of a pair of glasses (100) and a characteristic point (OG) of a head (10) of a wearer (1) of said pair of glasses (100), said characteristic plane (P1) of the pair of glasses (100) being substantially parallel to the average plane of the contours of lenses (150G, 150D) of the pair of glasses (100), said device (200) comprising:

- at least one light source (220) designed to illuminate the head (10) of the wearer (1),
- an image sensor (210) having an optical axis (A2) substantially directed towards the head (10) of the wearer (1) and designed to acquire at least two images (301, 302, 303) of the head (10) of the wearer (1) in which the pair of glasses (100) illuminated by the light source (220) appears and in which the head (10) of the wearer (1) has different inclinations about a pivoting axis (A1) substantially parallel to said characteristic plane (P1) and transverse to the optical axis (A2) of the image sensor (210), and
- a computing unit (250) designed to process said images (301, 302, 303),

**characterized in that** said computing unit (250) is designed to:

- determine, for each acquired image (301, 302, 303), an angle of inclination ($\alpha_{301}$, $\alpha_{302}$, $\alpha_{303}$) of the head (10) of the wearer with respect to the image sensor (210), measured between the optical axis (A2) of the image sensor (210) and a plane (P2) attached to the head (10) of the wearer (1) that is substantially parallel to said pivoting axis (A1) and substantially orthogonal to said characteristic plane (P1),
- acquire at least two predetermined simulated values (VG1, VG2, VG3) of the sought eye-to-glasses distance,
- iteratively compute, for each acquired image (301, 302, 303) and with each acquired simulated value (VG1, VG2, VG3), a morphological distance ($EG1_{301}$, $EG2_{301}$, $EG3_{301}$, $EG1_{302}$, $EG2_{302}$, $EG3_{302}$, $EG1_{303}$, $EG2_{303}$, $EG3_{303}$) between the characteristic point (OG) of the head (10) of the wearer (1) and the plane (P2) attached to the head (10) of the wearer (1), taking into account said angle of inclination ($a_{301}$, $\alpha_{302}$, $\alpha_{303}$), and

- select, from among the simulated values (VG1, VG2, VG3) of the eye-to-glasses distance, the value closest to the real eye-to-glasses distance (VG) of the wearer, on the basis of the computed morphological distances ($EG1_{301}$, $EG2_{301}$, $EG3_{301}$, $EG1_{302}$, $EG2_{302}$, $EG3_{302}$, $EG1_{303}$, $EG2_{303}$, $EG3_{303}$), said selection being made by determining, for each simulated value (VG1, VG2, VG3), a dispersion rate of the morphological distances associated with this simulated value (VG1, VG2, VG3), the selected simulated value (VG2) being the one for which the morphological distances are the least dispersed.

Fig.1

Fig.2

Fig.3

## Fig.4

Fig.5

Fig.6

Fig.7

Fig.8

**EP 2 822 450 B1**

## RÉFÉRENCES CITÉES DANS LA DESCRIPTION

**Documents brevets cités dans la description**

- WO 2008132356 A